# EUROPÄISCHE PATENTSCHRIFT

(11) **EP 2 797 667 B1**
(45) Veröffentlichungstag und Bekanntmachung des Hinweises auf die Patenterteilung: **28.09.2016**
(21) Anmeldenummer: 13704912.8
(22) Anmeldetag: 07.02.2013
(51) Int. Cl.: A61N 1/36, A61B 5/04

(54) **VORRICHTUNG ZUR EICHUNG EINER NICHT-INVASIVEN DESYNCHRONISIERENDEN NEUROSTIMULATION**
APPARATUS FOR CALIBRATING NON-INVASIVE DESYNCHRONIZING NEUROSTIMULATION
DISPOSITIF D'ÉTALONNAGE D'UNE NEUROSTIMULATION DÉSYNCHRONISANTE NON EFFRACTIVE

(30) Priorität: 08.02.2012 DE 102012002436
(43) Veröffentlichungstag der Anmeldung: 05.11.2014
(73) Patentinhaber: Forschungszentrum Jülich GmbH, 52425 Jülich (DE)
(72) Erfinder: TASS, Peter Alexander, 52428 Jülich (DE)
(74) Vertreter: Manitz, Finsterwald & Partner GbR
(86) Internationale Anmeldenummer: PCT/EP2013/052450
(87) Internationale Veröffentlichungsnummer: WO 2013/117655

(56) Entgegenhaltungen:
- WO-A1-2004/093981
- DE-A1- 10 233 960
- DATABASE INSPEC [Online] THE INSTITUTION OF ELECTRICAL ENGINEERS, STEVENAGE, GB; Juni 2011 (2011-06), LYSYANSKY B ET AL: "Desynchronizing Anti-resonance Effect of m: n ON-OFF coordinated reset stimulation", XP002703594, Database accession no. 11968959
- TASS P A: "Desynchronizing double-pulse phase resetting and application to deep brain stimulation", BIOLOGICAL CYBERNETICS, SPRINGER VERLAG. HEIDELBERG, DE, Bd. 85, Nr. 5, 1. November 2001 (2001-11-01), Seiten 343-354, XP002245895, ISSN: 0340-1200, DOI: 10.1007/S004220100268

## Beschreibung

Die Erfindung betrifft eine Vorrichtung zur Eichung einer nicht-invasiven desynchronisierenden Neurostimulation.

Bei Patienten mit neurologischen oder psychiatrischen Erkrankungen, z.B. Morbus Parkinson, essentiellem Tremor, Tinnitus, Dystonie oder Zwangserkrankungen, sind Nervenzellverbände in umschriebenen Bereichen des Gehirns krankhaft, z.B. übersteigert synchron, aktiv. In diesem Fall bildet eine große Anzahl von Neuronen synchron Aktionspotentiale aus, d.h., die beteiligten Neuronen feuern übermäßig synchron. Beim Gesunden hingegen feuern die Neuronen in diesen Hirngebieten qualitativ anders, z.B. auf unkorrelierte Weise.

Zur Behandlung derartiger Erkrankungen wurden Stimulationstechniken entwickelt, die gezielt krankhaft synchroner neuronaler Aktivität entgegenwirken. Insbesondere die "Coordinated Reset" (CR)-Stimulation zeichnet sich hierbei durch große therapeutische Wirksamkeit und Sicherheit aus (vgl. z.B. "A model of desynchronizing deep brain stimulation with a demand-controlled coordinated reset of neural subpopulations" von P. A. Tass, erschienen in Biol. Cybern. 89, 2003, Seiten 81 bis 88). Die CR-Stimulation kann mit unterschiedlichen Reizmodalitäten, z.B. mittels elektrischer Stimulation oder sensorischer, z.B. akustischer Stimulation realisiert werden. Besonders aussichtsreich, da deutlich nebenwirkungsärmer und kostengünstiger (und damit für eine größere Anzahl von Patienten zugänglich) sind die nicht-invasiven CR-Stimulationsverfahren und -vorrichtungen.

Wichtig für die Wirksamkeit der CR-Stimulation ist, dass die verschiedenen Orte im Gehirn oder Rückenmark, die durch die Stimulation gereizt werden, in der zu stimulierenden Neuronenpopulation (bzw. bei invasiver CR-Stimulation im zu stimulierenden Faserbündel) liegen. Bei invasiver CR-Stimulation wird die optimale Lokalisation der implantierten Elektrode im Rahmen der OP-Planung u.a. über detaillierte anatomische Informationen, z.B. aus kernspintomographischen Untersuchungen, sichergestellt.

Bei den nicht-invasiven Stimulationsverfahren hingegen ist die Wahl der optimalen Stimulationsorte im Gehirn oder Rückenmark, die einer Eichung von Stimulationsparametern, z.B. den Tonhöhen der Therapietöne bei akustischer CR-Stimulation, bzw. einer Eichung der Lokalisation der verschiedenen nicht-invasiven Aktoren (z.B. der Platzierung der vibro-taktilen Stimulatoren auf der Haut in Relation zum betroffenen Körperteil) entspricht, ein bis jetzt ungelöstes Problem. Zeitaufwändiges Probieren garantiert die optimale Wirksamkeit der nicht-invasiven CR-Therapie nicht, da einerseits nicht alle möglichen Stimulationsorte im Gehirn systematisch erschlossen und getestet werden, und andererseits die Patienten durch lange Untersuchungen strapaziert werden, so dass die Mitarbeit der Patienten naturgemäß leidet, und die Ergebnisse der Testung schlechter werden.

Aus dem Dokument DE 102 33 960 A1 ist eine herkömmliche Vorrichtung zur Stimulation von Neuronen mit einer krankhaft synchronen und oszillatorischen neuronalen Aktivität bekannt. Die Vorrichtung umfasst eine nicht-invasive Stimulationseinheit zur Applikation von Reizen an einen Patienten, wobei die Reize Neuronen im Gehirn und/oder Rückenmark des Patienten stimulieren, eine Messeinheit zum Aufnehmen von Messsignalen, die eine neuronale Aktivität der stimulierten Neuronen wiedergeben, und eine Steuer- und Analyseeinheit zur Steuerung der Stimulationseinheit und zur Analyse der Messsignale. Eine weitere Stimulationsvorrichtung ist in dem Dokument B. Lysyansky et al.: "Desynchronizing anti-resonance effect of m:n ON-OFF coordinated reset stimulation", J. Neural Eng. 8 (2011) 036019 (13pp) beschrieben.

Der Erfindung liegt die Aufgabe zugrunde, eine Vorrichtung anzugeben, die eine vom Untersucher unabhängige, automatisch durchgeführte, elektrophysiologisch basierte Eichung der Stimulationsparameter ermöglicht. Insbesondere soll diese Eichung ermöglichen, (i) die Therapie wirksam durchzuführen, (ii) Nebenwirkungen zu vermeiden, (iii) die zur Parametereinstellung durchzuführende Untersuchung möglichst kurz, praktikabel und für den Patienten erträglich zu gestalten.

Die der Erfindung zugrunde liegende Aufgabenstellung wird durch die Merkmale des Anspruchs 1 gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:
- Fig. 1: eine schematische Darstellung einer Vorrichtung zur nicht-invasiven desynchronisierenden Neurostimulation während des Betriebs;
- Fig. 2: ein Flussdiagramm zur Veranschaulichung der Eichung der in Fig. 1 dargestellten Vorrichtung;
- Fig. 3: eine schematische Darstellung einer Reizfolge zur Analyse der durch die Reize bewirkten Phasenrücksetzung;
- Fig. 4: eine schematische Darstellung einer EntrainmentReizfolge;
- Fig. 5: eine schematische Darstellung einer CR-Neuromodulation mit zwei gemäß Tonotopie bzw. Somatotopie benachbarten Reizen;
- Fig. 6: eine schematische Darstellung einer CR-Neuromodulation mit vier Reizen;
- Fig. 7: eine Darstellung von Sinusschwingungen mit verschiedenen Frequenzen;
- Fig. 8A und 8B: schematische Darstellungen verschiedener Töne und Tonpakete;
- Fig. 9A und 9B: schematische Darstellungen verschiedener akustischer CR-Neuromodulationen;
- Fig. 10: eine schematische Darstellung einer Vorrichtung zur akustischen desynchronisierenden Neurostimulation;
- Fig. 11: ein Flussdiagramm zur Veranschaulichung der Eichung der in Fig. 10 dargestellten Vorrichtung;
- Fig. 12: eine schematische Darstellung einer Vorrichtung zur optischen desynchronisierenden Neurostimulation;
- Fig. 13: eine schematische Darstellung des Gesichtsfelds eines Patienten;
- Fig. 14: eine schematische Darstellung einer Transmissionsbrille;
- Fig. 15: eine schematische Darstellung einer visuellen CR-Neuromodulation;
- Fig. 16: eine schematische Darstellung einer Vorrichtung zur taktilen, vibratorischen, thermischen und/oder elektrischen transkutanen desynchronisierenden Neurostimulation;
- Fig. 17A und 17B: schematische Darstellungen von vibratorischen Reizen;
- Fig. 18: eine schematische Darstellung eines taktilen Reizes;
- Fig. 19A und 19B: schematische Darstellungen von thermischen Reizen;
- Fig. 20: eine schematische Darstellung eines elektrischen transkutanen Reizes;
- Fig. 21: eine schematische Darstellung einer taktilen, vibratorischen, thermischen und/oder elektrischen transkutanen CR-Neuromodulation; und
- Fig. 22: ein Flussdiagramm zur Veranschaulichung der Eichung der in Fig. 16 dargestellten Vorrichtung.

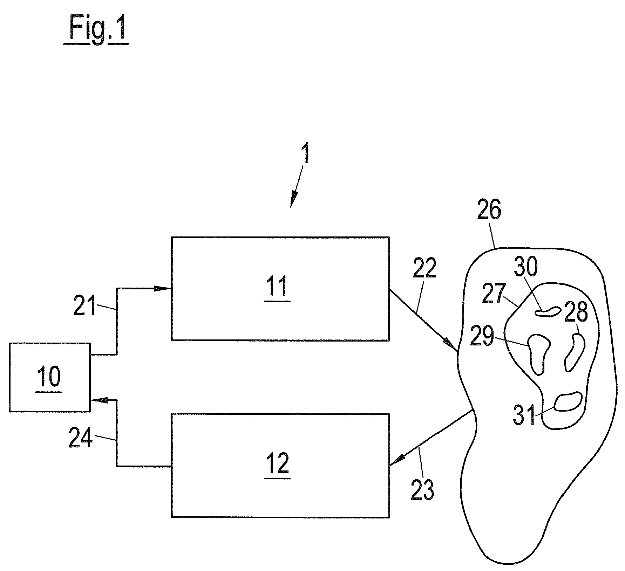

In Fig. 1 ist schematisch eine Vorrichtung 1 zur Eichung der Stimulationsparameter einer nicht-invasiven desynchronisierenden Neurostimulation dargestellt. Die Vorrichtung 1 besteht aus einer Steuer- und Analyseeinheit 10, einer Stimulationseinheit 11 und einer Messeinheit 12. Während des Betriebs der Vorrichtung 1 führt die Steuer- und Analyseeinheit 10 u.a. eine Steuerung der Stimulationseinheit 11 durch. Dazu erzeugt die Steuer- und Analyseeinheit 10 Steuersignale 21, die von der Stimulationseinheit 11 entgegengenommen werden. Die Stimulationseinheit 11 erzeugt anhand der Steuersignale 21 Reize 22, die einem Patienten verabreicht werden. Die Reize 22 können Reize aus der Gruppe von akustischen, optischen, taktilen, vibratorischen, thermischen und elektrischen transkutanen Reizen sein. Die Reize 22 können vom Patienten insbesondere bewusst wahrnehmbar sein. Die Stimulationseinheit 11 und insbesondere auch die Steuer- und Analyseeinheit 10 sind nicht-invasive Einheiten, d.h., während des Betriebs der Vorrichtung 1 befinden sie sich außerhalb des Körpers des Patienten und werden nicht operativ in den Körper des Patienten implantiert.

Der durch die Reize 22 erzielte Stimulationseffekt wird mit Hilfe der Messeinheit 12 kontrolliert. Die Messeinheit 12 nimmt ein oder mehrere am Patienten gemessene Messsignale 23 auf, wandelt diese gegebenenfalls in elektrische Signale 24 um und führt diese der Steuer- und Analyseeinheit 10 zu. Insbesondere kann mittels der Messeinheit 12 die neuronale Aktivität in dem stimulierten Zielgebiet oder einem mit dem Zielgebiet verbundenen Gebiet gemessen werden, wobei die neuronale Aktivität dieses Gebiets mit der neuronalen Aktivität des Zielgebiets hinreichend eng korreliert (z.B. Muskelaktivität). Die Steuer- und Analyseeinheit 10 verarbeitet die Signale 24, z.B. können die Signale 24 verstärkt und gefiltert werden, und analysiert die verarbeiteten Signale 24. Anhand der Ergebnisse dieser Analyse steuert die Steuer- und Analyseeinheit 10 insbesondere die Stimulationseinheit 11 an. Zur Durchführung ihrer Aufgaben kann die Steuer- und Analyseeinheit 10 beispielsweise einen Prozessor (z.B. einen Mikrocontroller) enthalten.

Die Messeinheit 12 enthält ein oder mehrere Sensoren, die es insbesondere ermöglichen, (i) eine Stimulus-induzierte Zurücksetzung der Phase der pathologischen oszillatorischen Aktivität und (ii) eine Ab- bzw. Zunahme der Amplitude der pathologischen oszillatorischen Aktivität nachzuweisen.

Als Sensoren können nicht-invasive Sensoren eingesetzt werden, z.B. Elektroenzephalographie (EEG)-Elektroden, Magnetenzephalographie (MEG)-Sensoren und Sensoren zur Messung lokaler Feldpotentiale (LFP). Die neuronale Aktivität kann auch indirekt durch Messung der damit einhergehenden Muskelaktivität mittels Elektromyographie (EMG) ermittelt werden.

Alternativ können die Sensoren in den Körper des Patienten implantiert sein. Als invasive Sensoren können beispielsweise epikortikale Elektroden, Tiefenhirnelektroden, sub- oder epidurale Hirnelektroden, subkutane EEG-Elektroden und sub- oder epidurale Rückenmarkselektroden dienen. Des Weiteren können an peripheren Nerven zu befestigende Elektroden als Sensoren eingesetzt werden.

Es kann durchaus vorgesehen sein, dass die einzelnen Komponenten der Vorrichtung 1, insbesondere die Steuer- und Analyseeinheit 10, die Stimulationseinheit 11 und/ oder die Messeinheit 12, baulich voneinander getrennt sind. Die Vorrichtung 1 kann daher auch als System aufgefasst werden.

Die Vorrichtung 1 kann insbesondere zur Behandlung von neurologischen oder psychiatrischen Erkrankungen eingesetzt werden, z.B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d.h., die beteiligten Neuronen feuern übermäßig synchron. Hinzu kommt, dass die kranke Neuronenpopulation eine oszillatorische neuronale Aktivität aufweist, d.h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z.B. auf unkorrelierte Weise.

In Fig. 1 ist die Vorrichtung 1 während einer CR-Stimulation dargestellt. Im Gehirn 26 oder Rückenmark 26 des Patienten weist mindestens eine Neuronenpopulation 27 eine wie vorstehend beschriebene krankhaft synchrone und oszillatorische neuronale Aktivität auf. Die Stimulationseinheit 11 verabreicht dem Patienten die Reize 22 derart, dass die Reize 22 je nach Modalität über die Augen, die Ohren oder die Haut des Patienten aufgenommen werden und von dort über das Nervensystem an die krankhaft aktive Neuronenpopulation 27 im Gehirn 26 und/oder Rückenmark 26 weitergeleitet werden. Die Reize 22 sind so ausgestaltet, dass die krankhaft synchrone Aktivität der Neuronenpopulation 27 desynchronisiert wird. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen.

Bei der Applikation akustischer (bzw. auditorischer) oder optischer (bzw. visueller) Reize 22 werden diese über mindestens ein Ohr bzw. mindestens ein Auge des Patienten aufgenommen. Die taktilen, vibratorischen, thermischen und elektrischen transkutanen Reize 22 (bzw. Tast-, Vibrations-, Thermo- und Elektroreize) werden von in oder unter der Haut gelegenen Rezeptoren aufgenommen und an das Nervensystem weitergeleitet. Zu diesen Rezeptoren zählen beispielsweise Merkel-Zellen, Ruffini-Körperchen, Meissner-Körperchen und Haarfollikelrezeptoren, die insbesondere als Rezeptoren für die taktilen Reize 22 wirken. Die vibratorischen Reize 22 zielen vorwiegend auf die Tiefensensibilität ab. Die vibratorischen Reize 22 können von in der Haut, den Muskeln, dem Subkutangewebe und/oder den Sehnen des Patienten gelegenen Rezeptoren aufgenommen werden. Als Rezeptoren für die vibratorischen Reize 22 seien beispielhaft die Vater-Pacini-Körperchen genannt, die Vibrationsempfindungen und Beschleunigungen vermitteln. Die thermischen Reize 22 werden von den Thermorezeptoren der Haut aufgenommen. Dies sind Warmrezeptoren (auch Wärmerezeptoren, Warmsensoren oder Wärmesensoren genannt) und Kaltsensoren (auch Kältesensoren, Kaltrezeptoren oder Kälterezeptoren genannt). In der Haut des Menschen liegen die Kaltsensoren mehr oberflächlich, die Warmrezeptoren etwas tiefer. Die elektrischen transkutanen Reize 22 wirken nicht spezifisch auf nur eine Gruppe von in oder unter der Haut gelegenen Rezeptoren. Mittels der elektrischen transkutanen Reize 22 kann das Zielgebiet daher über unterschiedliche Kanäle stimuliert werden.

Die gezielte Stimulation bestimmter Bereiche des Gehirns oder Rückenmarks wird durch die tonotope bzw. somatotope Zuordnung von Körperregionen zu diesen Bereichen ermöglicht. Beispielsweise werden akustische Reize im Innenohr in Nervenimpulse umgesetzt und über den Hörnerv zu dem auditorischen Cortex weitergeleitet. Durch die tonotope Anordnung des auditorischen Cortex wird bei der akustischen Stimulation des Innenohres mit einer bestimmten Frequenz ein bestimmter Teil des auditorischen Cortex aktiviert.

Bei der visuellen Stimulation werden unterschiedliche Stellen im Gesichtsfeld über die Linse des Auges auf unterschiedliche Stellen der Retina abgebildet. Die unterschiedlichen Stellen der Retina sind wiederum über den Sehnerv mit unterschiedlichen Neuronen im Gehirn verbunden. Folglich können mit den an unterschiedlichen räumlichen Orten applizierten Reizen jeweils unterschiedliche Neuronen stimuliert werden.

Aufgrund der somatotopen Gliederung der Nervenleitungsbahnen und zugehörigen Hirngebiete werden des Weiteren durch taktile, vibratorische, thermische und elektrische transkutane Reize, die an unterschiedlichen Stellen der Haut appliziert werden, unterschiedliche Neuronen stimuliert. Bei diesen Stimulationsformen können die Stimulationselemente beispielsweise am Fuß, Unterschenkel und Oberschenkel oder aber an der Hand, dem Unterarm und Oberarm des Patienten angebracht werden, um dadurch bestimmte Neuronen stimulieren zu können.

Die Stimulationseinheit 11 kann demnach unterschiedliche Bereiche des Gehirns 26 oder Rückenmarks 26 separat stimulieren, indem die applizierten Reize 22 über Nervenleitungen an unterschiedliche Zielgebiete, die im Gehirn 26 und/oder Rückenmark 26 liegen, weitergeleitet werden. Die Zielgebiete können während der CR-Stimulation mit eventuell unterschiedlichen und/oder zeitversetzten Reizen 22 stimuliert werden.

Bei der CR-Stimulation werden der Neuronenpopulation 27, die eine krankhaft synchrone und oszillatorische Aktivität aufweist, Reize 22 verabreicht, welche in der Neuronenpopulation 27 ein Zurücksetzen, einen sogenannten Reset, der Phase der neuronalen Aktivität der stimulierten Neuronen bewirken. Durch das Zurücksetzen wird die Phase der stimulierten Neuronen unabhängig von dem aktuellen Phasenwert auf einen oder nahe zu einem bestimmten Phasenwert, z.B. 0°, gesetzt (in der Praxis ist es nicht möglich, einen bestimmten Phasenwert exakt einzustellen, dies ist für eine erfolgreiche CR-Stimulation aber auch nicht erforderlich). Somit wird die Phase der neuronalen Aktivität der krankhaften Neuronenpopulation 27 mittels einer gezielten Stimulation kontrolliert. Da es ferner möglich ist, die krankhafte Neuronenpopulation 27 an unterschiedlichen Stellen zu stimulieren, kann die Phase der neuronalen Aktivität der krankhaften Neuronenpopulation 27 an den unterschiedlichen Stimulationsstellen zu unterschiedlichen Zeitpunkten zurückgesetzt werden. Im Ergebnis wird dadurch die krankhafte Neuronenpopulation 27, deren Neuronen zuvor synchron und mit gleicher Frequenz und Phase aktiv waren, in mehrere Subpopulationen aufgespalten, die in Fig. 1 schematisch dargestellt sind und mit den Bezugszeichen 28, 29, 30 und 31 gekennzeichnet sind (beispielhaft sind hier vier Subpopulationen dargestellt). Innerhalb einer der Subpopulationen 28 bis 31 sind die Neuronen nach dem Zurücksetzen der Phase weiterhin synchron und feuern auch weiterhin mit derselben pathologischen Frequenz, aber jede der Subpopulationen 28 bis 31 weist bezüglich ihrer neuronalen Aktivität die Phase auf, die ihr durch den Stimulationsreiz aufgezwungen wurde. Dies bedeutet, dass die neuronalen Aktivitäten der einzelnen Subpopulationen 28 bis 31 nach dem Zurücksetzen ihrer Phasen weiterhin einen in etwa sinusförmigen Verlauf mit derselben pathologischen Frequenz haben, aber unterschiedliche Phasen.

Bedingt durch die krankhafte Interaktion zwischen den Neuronen ist der durch die Stimulation erzeugte Zustand mit mindestens zwei Subpopulationen instabil, und die gesamte Neuronenpopulation 27 nähert sich schnell einem Zustand kompletter Desynchronisation, in welchem die Neuronen unkorreliert feuern. Der gewünschte Zustand, d.h. die komplette Desynchronisation, ist somit nach der zeitversetzten (oder phasenverschobenen) Applikation der phasenrücksetzenden Reize 22 nicht sofort vorhanden, sondern stellt sich meist innerhalb weniger Perioden oder gar in weniger als einer Periode der pathologischen Frequenz ein.

Eine Theorie zur Erklärung des Stimulationserfolgs basiert darauf, dass die letztlich gewünschte Desynchronisation durch die krankhaft gesteigerte Interaktion zwischen den Neuronen erst ermöglicht wird. Hierbei wird ein Selbstorganisationsprozess ausgenutzt, der für die krankhafte Synchronisation verantwortlich ist. Derselbe bewirkt, dass auf eine Aufteilung einer Gesamtpopulation 27 in Subpopulationen 28 bis 31 mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die CR-Stimulation eine Neuorganisation der Konnektivität der gestörten neuronalen Netzwerke erzielt werden, so dass lang anhaltende therapeutische Effekte bewirkt werden können. Der erzielte synaptische Umbau ist von großer Bedeutung für die wirksame Behandlung neurologischer oder psychiatrischer Erkrankungen.

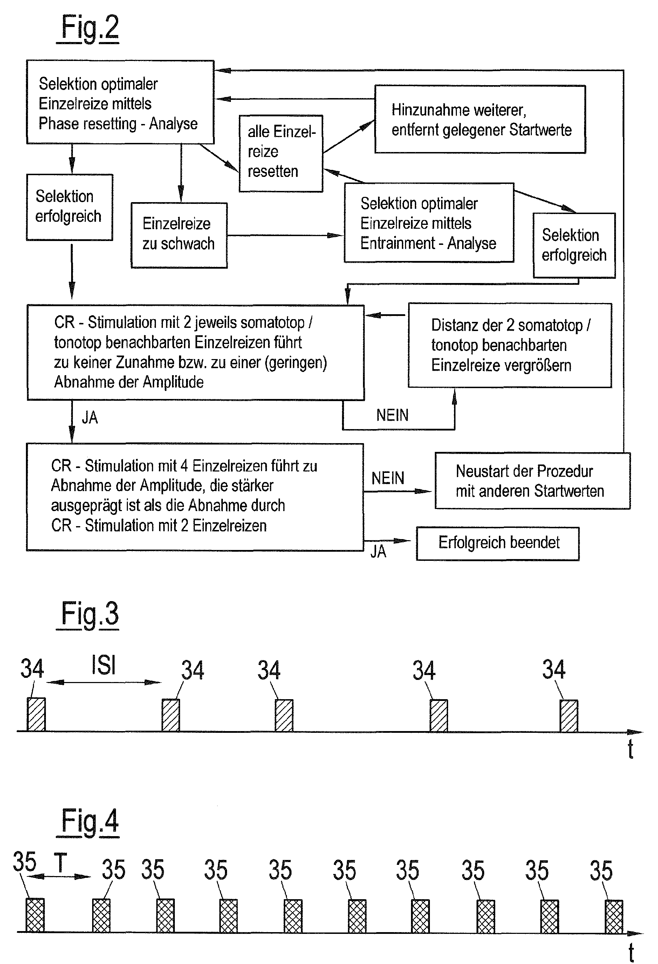

Im Folgenden wird die Eichung beschrieben, die mit der Vorrichtung 1 durchgeführt wird, um damit die optimalen Reizparameter für die nicht-invasive CR-Stimulation zu ermitteln. Die im Rahmen dieser Eichung durchgeführten Schritte sind im Flussdiagram von Fig. 2 zusammengefasst.

In einem ersten Schritt erzeugt die Stimulationseinheit 11 erste Reize 34 und verabreicht diese dem Patienten. Ziel dieses Schritts ist es, den synchronen Fokus im Gehirn bzw. Rückenmark zu lokalisieren. Die ersten Reize 34 können vorgegeben sein und stellen insbesondere eine Vorauswahl an möglichen Einzelreizen dar, deren Wirksamkeit bei der CR-Stimulation medizinisch plausibel ist. Beispielsweise können solche Reize als erste Reize 34 ausgewählt werden, mit denen bei einem anderen Patienten mit gleichem oder ähnlichem Krankheitsbild eine erfolgreiche CR-Stimulation durchgeführt werden konnte. Die ersten Reize 34 der Startauswahl weisen jeweils einen Reizparameter auf, der innerhalb eines ersten Reizparameterbereichs liegt.

Im Fall der akustischen Stimulation können beispielsweise Therapietöne in einem Intervall um die dominante Tinnitusfrequenz des Patienten (mit tonalem Tinnitus) als erste Reize 34 gewählt werden. In diesem Fall entspricht der Reizparameter folglich einer Frequenz und der erste Reizparameterbereich einem Frequenzintervall. Das Intervall von Therapietönen kann auch an die Ausdehnung der Hörsenke bzw. Hörminderung angepasst sein, d.h. diese einschließen. Bei Patienten mit nicht-tonalem Tinnitus (Rauschen oder Geräusche) kann ein Startwert des Intervalls von CR-Therapietönen von einer audiometrischen Vergleichsmessung (des Frequenzintervalls des Tinnitus) stammen. Es können aber auch jeweils für die in der Praxis typischen Arten von Ohrgeräuschen Standard-Intervalle von CR-Therapietönen gewählt werden.

Im Fall der optischen Stimulation wird ein bestimmter Bereich des Gesichtsfelds als erster Reizparameterbereich gewählt. Durch die Applikation optischer erster Reize 34 in diesem Gesichtsfeldbereich werden bestimmte Bereiche des Gehirns 26 stimuliert.

Im Fall der vibro-taktilen bzw. thermischen bzw. transkutanen elektrischen Stimulation wird ein Hautareal als erster Reizparameterbereich ausgewählt, welches das erkrankte Körperteil einschließt (also etwas größer ist, damit die tatsächlich benötigte Ausdehnung durch die Selektion der optimalen Einzelreize ermittelt werden kann) bzw. Repräsentationen (z.B. Headsche Zonen) des erkrankten Körperteils bzw. Organs einschließt. Innerhalb des ausgewählten Hautareals werden die ersten Reize 34 appliziert.

Das eindimensionale Startintervall von Tönen bzw. der zweidimensionale Gesichtsfeldbereich bzw. das zweidimensionale Startareal der Haut kann anschließend gemäß der dem Fachmann bekannten physiologischen Abbildungscharakteristika (z.B. in erster Näherung logarithmische tonotope Karte im primären auditorischen Cortex und analoge Verhältnisse z.B. bei der taktilen Hautreizung) so mit den ersten Reizen 34 abgedeckt werden, dass diese die jeweilige kortikale Repräsentation in erster Näherung äquidistant abdecken, d.h., die zugehörigen Zielorte im Gehirn oder Rückenmark, die durch die ersten Reize 34 stimuliert werden, sollen in erster Näherung gleiche räumliche Entfernungen voneinander haben, also äquidistant sein.

Die ersten Reize 34 werden anschließend von der Steuer- und Analyseeinheit 10 darauf getestet, ob sie die Phase der pathologischen, synchronisierten und oszillatorischen Hirnaktivität zurücksetzen (resetten) können. Dabei werden aus der Vorauswahl an ersten Reizen 34 diejenigen ersten Reize 34 selektiert, welche die Phase der pathologischen, synchronisierten und oszillatorischen Hirnaktivität (bzw. der Muskelaktivität) zurückzusetzen vermögen. Verfahren zur Überprüfung einer derartigen Phasenrücksetzung sind dem Fachmann bekannt.

Die Analyse der Phasenrücksetzung der synchronen neuronalen Aktivität erfolgt typischerweise mittels eines Ensembles von identischen ersten Reizen 34 (d.h. Einzelreizen). Beispielhaft ist in Fig. 3 ein solches Ensemble von ersten Reizen 34 gegen die Zeit t aufgetragen. Zur Vermeidung von "Entrainment"-Phänomenen sollte ein Interstimulus-Intervall ISI zwischen den einzelnen ersten Reizen 34 von hinreichend großer und randomisierter Länge eingehalten werden. Das mittlere Interstimulus-Intervall sollte im Vergleich zur eigentlichen Reizantwort lang genug sein, damit die Reizantworten nicht überlappen und bei Verabreichung des nachfolgenden Reizes vollständig abgeklungen sind.

Eine Möglichkeit, die dem Fachmann zur Analyse der Phasenrücksetzung geläufig ist, besteht aus einer "phase resetting"-Analyse, wie sie beispielsweise in dem Artikel "Stochastic phase resetting of two coupled phase oscillators stimulated at different times" von P. A. Tass (erschienen in Physical Review E 67, 2003, Seiten 051902-1 bis 051902-15) beschrieben ist. Dazu wird der "phase resetting"-Index ermittelt (vgl. Gleichung 8, "stimulus-locking index" für v = 1). Die hierbei zur Berechnung der Phasenrücksetzung verwandte Phase wird z.B. mittels der Hilbert-Transformation aus dem mittels Bandpassfilterung bzw. "empirical mode decomposition" bestimmten Signal, welches die pathologische oszillatorische Aktivität repräsentiert, ermittelt (letztere ermöglicht im Vergleich zur Bandpassfilterung eine parameterunabhängige Bestimmung physiologisch relevanter Moden in verschiedenen Frequenzbereichen, vgl. "The empirical mode decomposition and the Hilbert spectrum for nonlinear and nonstationary time series analysis" von N. E. Huang et al. (erschienen in Proceedings of the Royal Society of London Series A, 1998, Band 454, Seiten 903 bis 995); die Kombination "empirical mode decomposition" mit nachfolgender Hilbert-Transformation wird als Hilbert-Huang-Transformation bezeichnet, vgl. "A confidence limit for the empirical mode decomposition and Hilbert spectral analysis" von N. E. Huang et al. (erschienen in Proceedings of the Royal Society of London Series A, 2003, Band 459, Seiten 2317 bis 2345). Eine Phasenrücksetzung ist erreicht, wenn der "phase resetting"-Index die 99. Perzentile der prä-Stimulus-Verteilung des "phase resetting"-Index überschreitet (vgl. Fig. 4 in "Stochastic phase resetting of two coupled phase oscillators stimulated at different times" von P. A. Tass). Falls medizinisch eine stärkere phasenrücksetzende Wirkung wünschenswert ist, können auch höhere Schwellen, z.B. das Doppelte oder das Dreifache der 99. Perzentile der prä-Stimulus-Verteilung des "phase resetting"-Index gewählt werden.

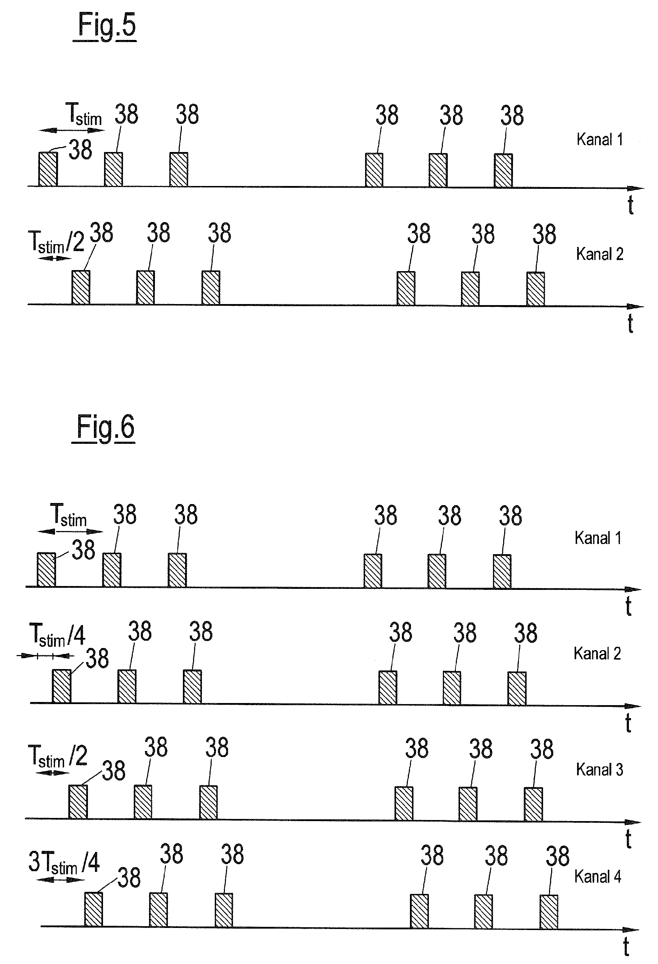

Alternativ zu dieser Datenanalyse können auch einfachere Datenanalyse-Verfahren angewandt werden, die die Detektion der Phasenrücksetzung mit in der Praxis hinreichender Genauigkeit zu approximieren vermögen. Z.B. kann einfach über das Ensemble an Reizantworten gemittelt werden. Von einer Phasenrücksetzung ist dann approximativ auszugehen, wenn der maximale Betrag der Reizantwort die 99. Perzentile der prä-Stimulus-Verteilung der gemittelten Antwort (oder deren Doppeltes bzw. Dreifaches) überschreitet (vgl. Fig. 6 in "Stochastic phase resetting of two coupled phase oscillators stimulated at different times" von P. A. Tass).

Sollten die ersten Reize 34 zu schwach sein, d.h., eine Phasenrücksetzung auch bei (gemäß physiologischen Kriterien) mittlerer Intensität der Einzelreize nicht zu erzielen sein, kann eine weiche Phasenrücksetzung ("soft phase reset") durchgeführt werden, die dem Fachmann beispielsweise aus dem Artikel "Desynchronization of brain rhythms with soft phase-resetting techniques" von P. A. Tass (erschienen in Biol. Cybern. 87, 2002, Seiten 102 bis 115) bekannt ist. Dabei appliziert die Stimulationseinheit 11 eine in Fig. 4 schematisch dargestellte periodische Folge von Einzelreizen 35, wobei die Periode T der periodischen Folge von Einzelreizen 35 typischerweise (und idealerweise) nahe bei der mittleren Periode der pathologischen Oszillation liegt. Beispielsweise kann ein Literaturwert für die mittlere Periode der pathologischen Oszillation herangezogen werden, und von dem Literaturwert kann z.B. um bis zu ± 5%, ± 10% oder ± 20% abgewichen werden.

Das Prinzip der weichen Phasenrücksetzung beruht darauf, dass eine Oszillation nach dem sogenannten "Entrainment" (Mitführung) und der Beendung der entrainenden Reizabfolge eine Vorzugsphasendifferenz zur entrainenden Reizabfolge aufweist. Am einfachsten lässt sich dies dadurch nachweisen, dass man eine periodische Folge von z.B. 100 Einzelreizen 35 für das Entrainment appliziert, die ersten 10 Einzelreize 35 zur Vermeidung möglicher Transienten vernachlässigt und für die Folge der restlichen 90 Einzelreize 35 einen Entrainment-Index berechnet. Hierzu berechnet man den "phase locking"-Index (aus "Detection of n:m phase locking from noisy data: application to magnetoencephalography" von P. Tass et al.; erschienen in Physical Review Letters, 1998, Band 81, Nr. 15, Seiten 3291 bis 3294) zwischen der Phase der Folge der Einzelreize 35 (deren Phase innerhalb jeder Periode linear um 2n ansteigt) und der z.B. mittels Hilbert-Transformation bestimmten Phase des mittels Bandpassfilterung oder "empirical mode decomposition" gewonnenen Signals, welches die pathologische, synchrone neuronale Aktivität repräsentiert. Alternativ kann der "phase locking"-Index auch mittels der Berechnung des Betrags des zirkulären Mittelwertes aller Phasendifferenzen innerhalb der Folge der restlichen 90 Einzelreize 35 berechnet werden. Der "phase locking"-Index berechnet, wie stark die Verteilung der Phasendifferenzen innerhalb der Folge der restlichen 90 Einzelreize 35 von einer Gleichverteilung abweicht. Ein relevantes Entrainment liegt vor, wenn der "phase locking"-Index die 99. Perzentile (oder deren Doppeltes oder Dreifaches) der prä-Stimulus-Verteilung des "phase locking"-Index überschreitet. Die prä-Stimulus-Baseline des "phase locking"-Index wird berechnet, indem man vor der Reizapplikation eine Surrogatfolge von 90 Einzelreizen verwendet, um für das spontane (d.h. durch Stimulation unbeeinflusste) pathologische Signal ein "phase locking"-Index zu berechnen (also so tut, als würde ein Entrainment stattfinden, um zu sehen, welche Werte der "phase locking"-Index zufälligerweise annehmen kann). Diese Berechnung des "phase locking"-Index wird z.B. in 100 prä-Stimulus-Intervallen (jeweils mit der Länge von 90 Perioden der tatsächlich verwandten Einzelreize) durchgeführt. Dies liefert eine Verteilung, die das Ausmaß zufällig hoher Werte des "phase locking"-Index abschätzen lässt.

Sollte auch auf diese Weise ein Entrainment nicht möglich sein, kann die gesamte Prozedur mit einer neuen Startauswahl der ersten Reize 34 (z.B. anderes Tonintervall oder anderes Hautareal) neu gestartet werden.

Sofern alle ersten Reize 34 (oder 35) aus der Startauswahl (egal ob mittels "phase resetting"-Index oder Entrainment-Index bestimmt) eine Phasenrücksetzung erzielt haben, fügt die Steuer- und Analyseeinheit 10 der Startauswahl der ersten Reize 34 (oder 35) des ersten Reizparameterbereichs in einem zweiten Schritt zweite Reize aus einem zweiten Reizparameterbereich hinzu. Die zweiten Reize sind sogenannte "Randreize", die außerhalb des ersten Reizparameterbereichs liegen (z.B. Töne außerhalb des initialen Tonintervalls oder Lokalisationen außerhalb des initialen Gesichtsfeldbereichs oder Hautareals). Insbesondere umfasst der zweite Reizparameterbereich den ersten Reizparameterbereich, d.h., der erste Reizparameterbereich ist vollständig in dem zweiten Reizparameterbereich enthalten.

Auch die zweiten Reize werden wie oben beschrieben darauf getestet, ob sie die Phase der pathologischen, synchronisierten und oszillatorischen Hirnaktivität zurücksetzen können. Sollte dies nicht möglich sein, werden neue zweite Reize ausgewählt.

Die hierdurch erzielte Startauswahl soll die initiale Startauswahl der ersten Reize 34 (oder 35) umfassen und darüber hinaus noch, gemäß der tonotopen oder somatotopen Organisation des zugehörigen Hirnareals, weiter entfernte zweite Reize enthalten. Für die Selektion der wirksamen, d.h. phasenrücksetzenden Einzelreize ist es wichtig, die Grenzen des Tonintervalls bzw. bzw. des Gesichtsfeldbereichs bzw. des Hautareals auszuloten, um das Hirngebiet mit der pathologischen neuronalen Synchronisation möglichst vollständig mit den Einzelreizen abzudecken.

Gemäß einer Ausgestaltung erfolgt die Ermittlung der zweiten Reize nur, wenn bei einem Krankheitsbild gemäß der tonotopen oder somatotopen Organisation des zugehörigen, betroffenen Hirnareals davon auszugehen ist, dass nur ein umschriebener Bereich von Einzelreizen wirksam (bzw. besonders wirksam) sein kann.

Eine Ermittlung der zweiten Reize ist z.B. in den im Folgenden aufgeführten Fällen durchzuführen bzw. erlässlich.

Die akustische CR-Stimulation zur Behandlung des Tinnitus ist am wirksamsten mit Einzelreizen einer Tonhöhe, die in der Lage sind, die Phase der pathologisch synchronen Aktivität, die sich in einem umschriebenen Frequenzbereich des tonotop angeordneten zentralen auditorischen Systems befindet, zurückzusetzen. Hier sollte die Ermittlung der zweiten Reize wie oben beschrieben durchgeführt werden.

Die vibro-taktile CR-Stimulation, z.B. zur Behandlung von Bewegungsstörungen (Parkinson, Dystonie), pathologischem Tremor, Funktionsstörungen nach Schlaganfall, chronischen Schmerzsyndromen (auch Amputationsschmerz), ist am wirksamsten mit an Haut-Lokalisationen applizierten Einzelreizen, die in der Lage sind, die Phase der pathologisch synchronen Aktivität, die sich in einem umschriebenen Bereich des somatotop angeordneten zentralen sensomotorischen Systems befindet, zurückzusetzen. Hier sollte die Ermittlung der zweiten Reize wie oben beschrieben durchgeführt werden.

Bei ADHS und Zwangserkrankungen liegt der primäre Fokus der pathologisch synchronen neuronalen Aktivität nicht in primären sensorischen Hirnarealen, z.B. im auditorischen Cortex, oder im primären motorischen Cortex. Somit ist die CR-Stimulation nicht auf eine Desynchronisation spezieller Teilbereiche der zugehörigen primären sensorischen bzw. motorischen Hirnareale zu begrenzen. Vielmehr wird der desynchronisierende Effekt der CR-Stimulation an die nachgeschalteten Hirnareale, die pathologisch synchrone neuronale Aktivität aufweisen, weitergeleitet und entfaltet dort seine desynchronisierende Wirkung. Bei Erkrankungen, bei denen primär sensorische oder primär motorische Hirnareale nicht primär von der pathologischen neuronalen Synchronisation betroffen sind, kann auf die Ermittlung der zweiten Reize verzichtet werden. In diesen Fällen ist es aber umso wichtiger, die Wirksamkeit der CR-Stimulation gemäß dem weiter unten beschriebenen vierten Schritt zu testen, um z.B. eine zu kleine Streuung der Startwerte (die zu einer Desynchronisation in einem zu kleinen Hirnareal und damit zu einer verzögerten CR-Wirkung führt) ausschließen zu können.

Die zur Durchführung der hier beschriebenen Eichung von der Messeinheit 12 aufgenommenen Messsignale 23 werden z.B. entweder nach entsprechender Vorverarbeitung (z.B. dem Fachmann geläufige Elimination von Artefakten wie Blinzelartefakte) direkt anhand von EEG- bzw. MEG-Signalen oder aber nach Ermittlung der zugrunde liegenden Hirnströme mittels dem Fachmann bekannten Verfahren für die Rückwärtsrechnung (mittels räumlich verteilten Stromdichten oder mehreren Dipolen) analysiert. In letzterem Fall wird der Zeitgang von Hirnströmen bzw. Dipolmomenten analysiert. Dies ermöglicht eine spezifisch auf die phasenrücksetzende Wirkung in einem oder mehreren besonders relevanten Hirnarealen (z.B. dem primären auditorischen Cortex bei akustischer CR-Neuromodulation zur Behandlung des Tinnitus) abgestimmte Eichung durchzuführen.

Sollte der Bereich der wirksamen, d.h. phasenrücksetzenden ersten Reize 34 und eventuell zweiten Reize nicht zusammenhängend sein, sondern "Löcher" enthalten (z.B. kann inmitten des Intervalls der wirksamen Töne ein Ton unwirksam sein, d.h. keine Phasenrücksetzung bewirken), werden über eine feinere Rasterung der Startwerte der ersten Reize 34 die unterschiedlichen wirksamen (phasenrücksetzenden) Bereiche (z.B. Tonintervalle) ermittelt. Für die Behandlung kann dann separat für jeden wirksamen Bereich (z.B. jedes wirksame Tonintervall) eine Gruppe von für die CR-Stimulation wirksamen Einzelreizen wie oben und im Folgenden beschrieben ermittelt werden. Es können aber auch kombinierte CR-Tonsequenzen zur Behandlung verwandt werden.

Nachdem in den oben beschriebenen ersten beiden Schritten die Selektion der wirksamen, d.h. phasenrücksetzenden ersten und eventuell zweiten Einzelreize durchgeführt wurde, ermittelt die Steuer- und Analyseeinheit 10 in einem dritten Schritt diejenigen Einzelreize (im Folgenden als dritte Reize 38 bezeichnet), die das zugehörige Hirnareal möglichst äquidistant reizen, um zu vermieden, dass dieselbe Subpopulation über mehrere Kanäle stimuliert wird, was zu einer Anregung der synchronen Aktivität führen könnte. Dies wird durch folgendes funktionale Kriterium abgeschätzt. Verteilt auf den gesamten Bereich der wirksamen ersten und eventuell zweiten Reize werden neben den außen gelegenen ersten oder zweiten Reizen (z.B. den höchsten und tiefsten phasenrücksetzenden Tönen) gemäß dem Fachmann bekannter physiologischer Skalierung (z.B. logarithmische tonotope Skala) und gemäß medizinischer Praktikabilität (z.B. bei der Platzierung von vibro-taktilen Stimulatoren oder Thermostimulatoren) noch wenige weitere, im wirksamen Bereich liegende dritte Reize 38 ausgewählt. Fig. 5 zeigt schematisch eine CR-Stimulation, die mit zwei gemäß Tonotopie bzw. Somatotopie benachbarten dritten Reizen 38 durchgeführt wird, wobei jedem der beiden herangezogenen dritten Reize 38 ein Kanal entspricht. In jedem der beiden Kanäle wird der jeweilige dritte Reiz 38 in einer Sequenz periodisch mit der Periode Tₛₜᵢₘ appliziert. In dem vorliegenden Fall umfasst jede Sequenz drei dritte Reize 38, die Sequenzen können allerdings auch weitere dritte Reize 38 enthalten. Nach jeder Sequenz wird eine bestimmte Pause eingehalten und die Sequenz danach wiederholt. Ferner beträgt die zeitliche Verzögerung zwischen den Sequenzen unterschiedlicher Kanäle Tₛₜᵢₘ/2.

Die Periode Tₛₜᵢₘ wird nahe bei der mittleren Periode der pathologischen Oszillation gewählt. Beispielsweise wird die Stimulationsfrequenz fₛₜᵢₘ = 1/Tₛₜᵢₘ entweder angepasst an das zu desynchronisierende Frequenzband gewählt (z.B. bei pathologischer Synchronisation im Deltaband eine darin bzw. noch besser in der unteren Hälfte befindliche Stimulationsfrequenz, also z.B. 1,5 Hz) oder - z.B. vor Beginn jeder Testung (der jeweiligen Reize) quasi online - an den Peak im Powerspektrum des pathologischen Frequenzbands angepasst. In letzterem Fall wird die Stimulationsfrequenz fₛₜᵢₘ so gewählt, dass diese 1:1 der Peak-Frequenz bzw. einem kleineren n:m-Vielfachen derselben (falls die Stimulationsfrequenz sonst zu hoch, z.B. über 2 Hz läge, und damit psychoakustisch zu unangenehm wäre) entspricht (n, m sind ganze Zahlen). Ferner kann ein Literaturwert für die mittlere Periode der pathologischen Oszillation herangezogen werden, und die für die Stimulation verwendete Periode Tₛₜᵢₘ kann von diesem Literaturwert um z.B. bis zu ± 5%, ± 10% oder ± 20% abweichen. Typischerweise liegt die Stimulationsfrequenz fₛₜᵢₘ im Bereich von 1 bis 30 Hz.

Die dritten Reize 38 sind funktional gut gewählt, wenn Kombinationen von zwei gemäß Tonotopie bzw. Somatotopie benachbarter dritter Reize 38 als CR-Stimulation appliziert bewirken, dass kein Amplitudenanstieg der pathologischen Oszillation (also keine Verstärkung der Synchronisation der das Signal generierenden pathologisch synchronisierten Neuronenpopulation) oder sogar eine leichte Abnahme (entspricht einer schwachen Desynchronisation) auftritt. Sollte dies bei einem Paar nicht der Fall sein, so muss die (tonotope bzw. somatotope) Distanz zwischen den beiden dritten Reizen 38 vergrößert werden, z.B. indem der innere Einzelreiz vom äußeren Einzelreiz weggeschoben wird. Für die jeweils angrenzenden weiteren Nachbar-Einzelreize muss dieser Test dann nochmal durchgeführt werden. Der dritte Schritt wird auf alle Paare von tonotop bzw. somatotop benachbarten Einzelreizen angewandt, bis die dritten Reize 38 gefunden sind, von denen jeweils alle tonotop bzw. somatotop benachbarten Einzelreizpaare als CR-Stimulation appliziert keinen Amplitudenanstieg der pathologischen Oszillation bewirken.

In einem vierten Schritt prüft die Steuer- und Analyseeinheit 10, ob bei Verwendung aller im dritten Schritt bestimmten dritten Reizen 38 die zugehörige CR-Stimulation die krankhaft synchrone und oszillatorische neuronale Aktivität der stimulierten Neuronen unterdrückt und insbesondere desynchronisierend wirkt. Fig. 6 zeigt ein Beispiel mit insgesamt vier dritten Reizen 38, d.h. vier Kanälen. In jedem der vier Kanäle wird der jeweilige dritte Reiz 38 in einer Sequenz periodisch mit der Periode Tₛₜᵢₘ appliziert, wobei Tₛₜᵢₘ auch hier nahe bei der mittleren Periode der pathologischen Oszillation liegt bzw. um bis zu ± 5%, ± 10% oder ± 20% von dem Literaturwert abweicht (typischerweise liegt fₛₜᵢₘ = 1/Tₛₜᵢₘ im Bereich von 1 bis 30 Hz). In dem vorliegenden Beispiel umfasst jede Sequenz drei dritte Reize 38, die Sequenzen können allerdings auch mehr dritte Reize 38 enthalten. Nach jeder Sequenz wird eine bestimmte Pause eingehalten und die Sequenz danach wiederholt. Ferner beträgt die zeitliche Verzögerung zwischen den Sequenzen benachbarter Kanäle Tₛₜᵢₘ/4, da vier Kanäle vorhanden sind. Für den allgemeinen Fall von N Kanälen würde die zeitliche Verzögerung benachbarter Kanäle Tₛₜᵢₘ/N betragen.

Eine wie in Fig. 6 dargestellte CR-Stimulation mit allen selektierten dritten Reizen 38 sollte zu einer Abnahme der Amplitude des pathologischen Signals führen, was einer CR-induzierten Desynchronisation der zugrundeliegenden, krankhaft synchronisierten Neuronenpopulation entspricht. Sollte dies nicht der Fall sein, kann die gesamte Prozedur mit einer anderen Startauswahl der ersten Reize 34 erneut durchgeführt werden.

Ein typischer (applikativer) Fehler in der Handhabung der Vorrichtung 1 ist eine zu klein gewählte Startauswahl. Z.B. wird nur ein kleines Tonintervall oder nur ein kleines Hautareal ausgewählt. Der krankhafte synchrone neuronale Prozess ist aber deutlich ausgedehnter, so dass mit der gemäß Tonotopie bzw. Somatotopie zu kleinen Auswahl von Einzelreizen nur ein kleiner Teil der betroffenen Neuronenpopulation stimuliert und somit die Gesamtpopulation nicht effizient und schnell desynchronisiert werden kann.

Die bei der vorstehenden Eichprozedur verwendeten Einzelreize sollten eine Stärke nahe an der dem Fachmann bekannten Wahrnehmungsschwelle aufweisen, z.B. kann die Reizstärke bei der akustischen Stimulation nur wenige dB (z.B. 5 dB) oberhalb der Hörschwelle liegen. Die Reize können aber auch stärker gewählt werden. Darüber hinaus kann mit der Vorrichtung 1 die optimale Reizstärke (Reizintensität) kalibriert werden. Sie sollte natürlich stets in einem für den Patienten angenehmen Intensitätsbereich liegen; potentiell gesundheitsschädliche Reizintensitäten sollten gemieden werden.

Nach einer erfolgreichen Eichung kann die Therapie mittels CR-Neuromodulation durchgeführt werden. Dabei können unterschiedliche Arten der CR-Stimulation verwandt werden. Bei einer "N aus N"-CR-Stimulation werden pro Stimulationszyklus wie in Fig. 6 (für N = 4) alle N verschiedenen Einzelreize appliziert. Alternativ kann eine "M aus N"-CR-Stimulation (M < N) angewandt werden, d.h., pro Stimulationszyklus werden aus N verschiedenen Einzelreizen M verschiedene z.B. randomisiert selektiert und appliziert. Auf diese Weise kann durch eine größere Auswahl von Einzelreizen der psychophysische Eindruck der Stimulation verändert werden. D.h., akustische CR-Stimulationsfolgen können auf diese Weise komplexer klingen, so dass dem möglichen Eindruck der Monotonie beim Patienten vorgebeugt werden kann.

Durch die Vorrichtung 1 lässt sich die herkömmliche subjektive Messung (z.B. audiometrische Anpassung bei akustischer CR-Stimulation) bzw. klinische Austestung (z.B. bei der vibro-taktilen Stimulation oder Thermostimulation) durch eine objektive, systematisch durchzuführende Untersuchung ersetzen. Letztere ermöglicht, die Messung subjektiver Eindrücke des Patienten bzw. subjektiver Eindrücke des evaluierenden Arztes bzw. ärztlichen Hilfspersonals durch eine elektrophysiologisch basierte Messung der Reizantworten des Gehirns zur Eichung der optimalen Stimulationsparameter und Stimulationsorte zu ersetzen.

### Stimulationseinheiten zur Erzeugung akustischer Reize:

Im Folgenden werden Ausgestaltungen der nicht-invasiven Stimulationseinheit 11 zur Erzeugung akustischer Reize 22 beschrieben (die Reize 22 umfassen die ersten, zweiten und dritten Reize). Derartige Stimulationseinheiten lassen sich auch der deutschen Patentanmeldung Nr. 10 2008 015 259.5 mit dem Titel "Vorrichtung und Verfahren zur auditorischen Stimulation" entnehmen, die am 20. März 2008 beim Deutschen Patent- und Markenamt hinterlegt worden ist. Der gesamte Offenbarungsgehalt der deutschen Patentanmeldung Nr. 10 2008 015 259.5 wird hiermit in die Offenbarung der vorliegenden Anmeldung aufgenommen.

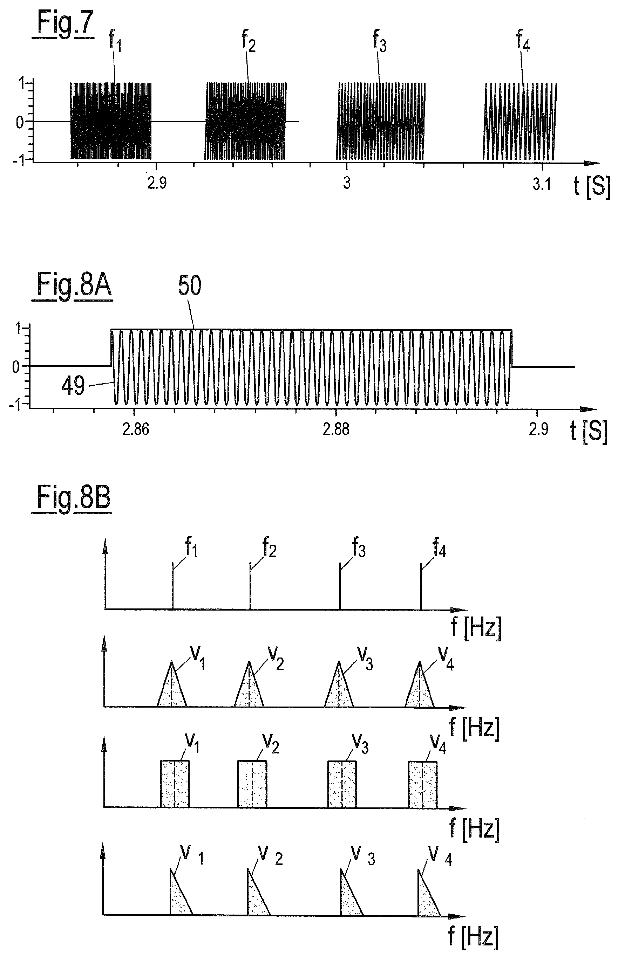

Das Frequenzspektrum der akustischen Reize kann ganz oder teilweise im für den Menschen hörbaren Bereich liegen. Vorzugsweise zur Behandlung von Patienten mit tonalem Tinnitus (oder auch zur Behandlung von Patienten mit rauschartigem Tinnitus) weisen die akustischen Reize beispielsweise die in Fig. 7 gezeigten vier reinen Töne mit den Frequenzen f₁, f2, f₃ und f₄ auf. Die akustischen Reize werden vom Patienten über ein oder beide Ohren aufgenommen, im Innenohr in Nervenimpulse umgesetzt und über den oder die Hörnerven an Neuronenpopulationen im Gehirn weitergeleitet. Die akustischen Reize sind derart ausgestaltet, dass sie Neuronenpopulationen im auditorischen Cortex stimulieren. Durch die tonotope Anordnung des auditorischen Cortex wird bei der akustischen Stimulation des Innenohres mit einer bestimmten Frequenz ein bestimmter Teil des auditorischen Cortex aktiviert. Die tonotope Anordnung des auditorischen Cortex ist z.B. in den folgenden Artikeln beschrieben: "Tonotopic organization of the human auditory cortex as detected by BOLD-FMRI" von D. Bilecen, K. Scheffler, N. Schmid, K. Tschopp und J. Seelig (erschienen in Hearing Research 126, 1998, Seiten 19 bis 27), "Representation of lateralization and tonotopy in primary versus secondary human auditory cortex" von D. R. M. Langers, W. H. Backes und P. van Dijk (erschienen in NeuroImage 34, 2007, Seiten 264 bis 273) und "Reorganization of auditory cortex in tinnitus" von W. Mühlnickel, T. Elbert, E. Taub und H. Flor (erschienen in Proc. Natl. Acad. Sci. USA 95, 1998, Seiten 10340 bis 10343).

Bei einer CR-Stimulation sind die akustischen Reize so ausgestaltet, dass mit ihnen beispielsweise die in Fig. 1 schematisch dargestellte Neuronenpopulation 27 des auditorischen Cortex mit einer krankhaft synchronen und oszillatorischen Aktivität stimuliert wird. Die Neuronenpopulation 27 lässt sich vor Beginn der Stimulation zumindest gedanklich in verschiedene Subpopulationen untergliedern, u.a. in die in Fig. 1 gezeigten Subpopulationen 28 bis 31. Vor Beginn der Stimulation feuern die Neuronen aller Subpopulationen 28 bis 31 weitgehend synchron und im Mittel mit der gleichen pathologischen Frequenz. Aufgrund der tonotopen Organisation des auditorischen Cortex werden mittels der Frequenz f₁ die Subpopulation 28, mittels der Frequenz f₂ die Subpopulation 29, mittels der Frequenz f₃ die Subpopulation 30 und mittels der Frequenz f₄ die Subpopulation 31 stimuliert. Die Stimulation mit den akustischen Reizen bewirkt in den jeweiligen Subpopulationen 28 bis 31 ein Zurücksetzen der Phase der neuronalen Aktivität der stimulierten Neuronen.

Aufgrund der tonotopen Anordnung des auditorischen Cortex sowie der Mehrzahl von Frequenzen f₁ bis f₄, die in den akustischen Reizen enthalten sind, ist es möglich, die krankhafte Neuronenpopulation 27 an den unterschiedlichen Stellen 28 bis 31 gezielt zu stimulieren. Dies ermöglicht es, die Phase der neuronalen Aktivität der krankhaften Neuronenpopulation 27 an den unterschiedlichen Stimulationsstellen 28 bis 31 zu unterschiedlichen Zeitpunkten zurückzusetzen, indem die Frequenzen f₁ bis f₄ zu unterschiedlichen Zeitpunkten appliziert werden. Im Ergebnis wird dadurch die krankhafte Neuronenpopulation 27, deren Neuronen zuvor synchron und mit gleicher Frequenz und Phase aktiv waren, in die Subpopulationen 28 bis 31 aufgespalten. Innerhalb jeder der Subpopulationen 28 bis 31 sind die Neuronen weiterhin synchron und feuern auch weiterhin im Mittel mit derselben pathologischen Frequenz, aber jede der Subpopulationen 28 bis 31 weist bezüglich ihrer neuronalen Aktivität die Phase auf, die ihr durch den Stimulationsreiz mit der zugehörigen Frequenz f₁ bis f₄ aufgezwungen wurde.

Bedingt durch die krankhafte Interaktion zwischen den Neuronen ist der durch die Stimulation erzeugte Zustand mit mehreren Subpopulationen 28 bis 31 instabil, und die gesamte Neuronenpopulation 27 nähert sich schnell einem Zustand kompletter Desynchronisation, in welchem die Neuronen unkorreliert feuern.

Um den auditorischen Cortex an unterschiedlichen Stellen fokal zu stimulieren, müssen reine Töne der zugehörigen Frequenzen f₁, f₂, f₃ und f₄ verabreicht werden. Infolge der tonotopen Anordnung des auditorischen Cortex werden unterschiedliche Teile des Gehirns durch die gleichzeitige Verabreichung der zugehörigen unterschiedlichen reinen Töne f₁ bis f₄, d.h. durch die Superposition verschiedener Sinusschwingungen stimuliert. Sollen die vier unterschiedlichen Orte 28 bis 31 z.B. zu unterschiedlichen Zeiten gereizt werden, werden die vier verschiedenen Frequenzen f₁ bis f₄ zu den jeweiligen Zeiten appliziert. Beispielhaft ist dies in Fig. 7 gezeigt. Hier werden Sinusschwingungen mit den Frequenzen f₁ = 1000 Hz, f₂ = 800 Hz, f₃ = 600 Hz und f₄ = 400 Hz sukzessive und pulsförmig appliziert, was zu einer sukzessiven fokalen Reizung an den vier verschiedenen Orten 28 bis 31 des auditorischen Cortex führt. Die Stärke der durch die jeweilige Sinusschwingung erzeugten Reizung des jeweiligen Areals im auditorischen Cortex entspricht der Amplitude der jeweiligen Sinusschwingung.

Die Generierung der in Fig. 7 gezeigten pulsförmigen Sinusschwingungen ist in Fig. 8A beispielhaft dargestellt. Dort wird eine Sinusschwingung 49 mit einer Rechteckfunktion 50, die beispielsweise die Werte 0 oder 1 annehmen kann, multipliziert. Zu den Zeitpunkten, zu denen die Rechteckfunktion 50 den Wert 0 hat, ist der zugehörige Reiz abgeschaltet und während der Zeit, in der die Rechteckfunktion 50 gleich 1 ist, ist der Reiz angeschaltet.

Anstelle der Rechteckfunktion 50 kann die Sinusschwingung 49 mit einer beliebigen anderen Funktion multipliziert werden. Im Ergebnis entspricht diese Multiplikation einer Amplitudenmodulation der Sinusschwingung 49. Um Klickgeräusche aufgrund eines scharfen Beginns und Endes der Töne zu vermeiden, kann statt der Rechteckfunktion 50 ein glatterer Verlauf gewählt werden, z.B. durch Multiplikation der Sinusschwingung 49 mit einer Sinus-Halbschwingung von geeigneter Dauer, z.B. der Dauer eines Reizes.

Anstelle der vorstehend beschriebenen Sinusschwingungen können auch oszillierende Signale mit einer anderen Signalform, wie z.B. Rechtecksignale, die mit der entsprechenden Grundfrequenz oszillieren, zur Generierung der akustischen Reize herangezogen werden.

Sofern statt einer fokalen Reizung eine weniger fokale Reizung durchgeführt werden soll, die größere Teile des auditorischen Cortex aktiviert, so werden Frequenzgemische anstelle von einzelnen Frequenzen, beispielsweise pulsförmig appliziert. Mittels eines Frequenzgemisches in den Grenzen zwischen einer unteren Frequenz fᵤₙₜₑₙ und einer höheren Frequenz f_{oben} werden all die Teile des auditorischen Cortex gereizt, die durch die Frequenzen zwischen funten und f_{oben} aufgrund der tonotopen Anordnung stimuliert werden. Sollen z.B. vier unterschiedliche größere Bereiche des auditorischen Cortex zu unterschiedlichen Zeiten stimuliert werden, so werden die vier zugehörigen Frequenzgemische mit den Grenzen f_{j,unten} und f_{j,oben} (j = 1, 2, 3, 4) zu den gewünschten Zeiten appliziert.

Zur Behandlung von Patienten mit rauschendem Tinnitus können statt der vier reinen Töne mit den Frequenzen f₁, f₂, f₃ und f₄ Tonpakete mit den jeweiligen Frequenzverteilungen v₁, v₂, v₃ und v₄ mit einer absoluten Breite (in Hz) oder einer relativen (d.h. zur Mittenfrequenz oder Frequenz maximaler Power normierten) Breite verwendet werden. Beispiele für derartige Frequenzverteilungen v₁, v₂, v₃ und v₄ finden sich in Fig. 8B.

Die oberste Teilabbildung zeigt die vorwiegend bei Patienten mit tonalem Tinnitus verwendeten reinen Töne f₁, f₂, f₃ und f₄. Bei Patienten mit rauschendem Tinnitus können auch Tonpakete verwendet werden. Hierbei kann es sich um zu einer Mittenfrequenz symmetrische Verteilungen (zweite und dritte Teilabbildung von oben) oder auch um asymmetrische Verteilungen (unterste Teilabbildung) handeln. Die Verteilungen können, wie in Fig. 8B dargestellt, Kanten aufweisen oder glatte Kurven darstellen. Die Phasen der Frequenzen der einzelnen Verteilung vⱼ können unter Verwendung unterschiedlicher, dem Fachmann bekannter Rauschprozesse randomisiert sein. Die Phasen der einzelnen Frequenzen können dabei wechselseitig unkorreliert sein, oder dem Fachmann bekannte (z.B. exponentiell mit der Frequenzdifferenz abfallende) Korrelationen aufweisen. Auch können die Phasen in den unterschiedlichen Frequenzverteilungen v₁, v₂, v₃ und v₄ miteinander korreliert oder unabhängig voneinander sein. Z.B. kann in allen Frequenzverteilungen v₁, v₂, v₃ und v₄ derselbe Rauschprozess die Phasen der Einzelfrequenzen bestimmen. Sowohl die Phasen der Einzelfrequenzen als auch die Frequenzverteilungen können aber auch dadurch ermittelt werden, dass dem Patienten ein Repertoire aus verschiedenen Frequenzverteilungen und Phasenverteilungen (z.B. unterschiedlichen Rauschprozessen) angeboten wird und die Frequenzverteilung und Phasenverteilung gewählt wird, deren Klangcharakteristik dem Ohrgeräusch des Patienten am ähnlichsten ist.

Anhand der vier reinen Töne mit den Frequenzen f₁ bis f₄ soll nachfolgend beispielhaft eine akustische CR-Stimulation erläutert werden, bei der durch zeitversetztes Zurücksetzen der Phasen der neuronalen Aktivität von Subpopulationen einer krankhaft synchronen und oszillatorischen Neuronenpopulation eine Desynchronisation der gesamten Neuronenpopulation erzielt werden kann. Die vier Frequenzen f₁ bis f₄ sind lediglich beispielhaft zu verstehen, d.h., es kann eine beliebige andere Zahl von Frequenzen oder Frequenzgemischen zu Stimulationszwecken eingesetzt werden. Beispielsweise kann die CR-Stimulation mit den in Fig. 8B gezeigten Tonpaketen mit den Frequenzverteilungen v₁ bis v₄ anstelle der vier reinen Töne mit den Frequenzen f₁ bis f₄ durchgeführt werden.

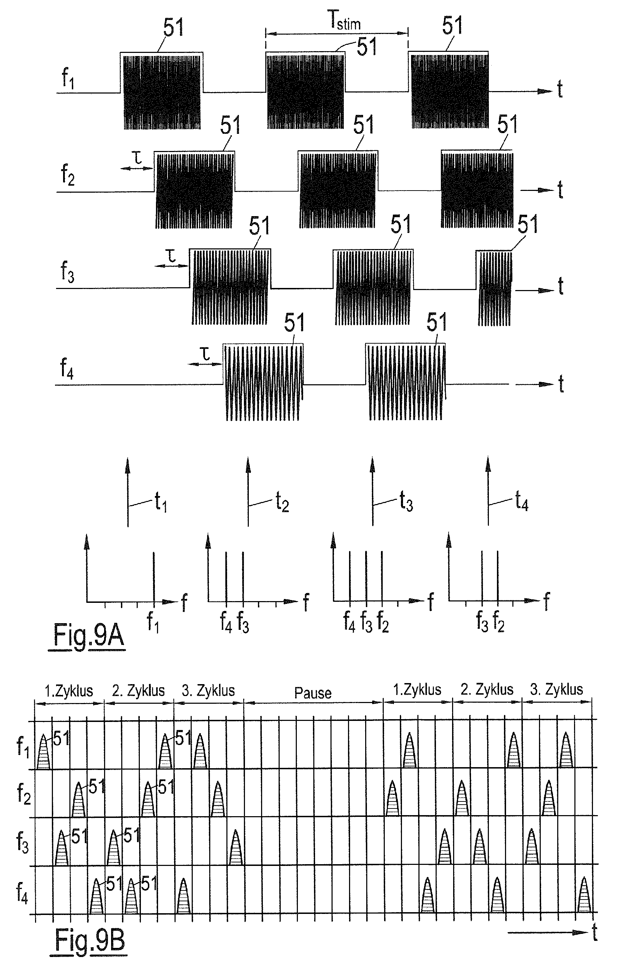

Ein für die oben beschriebenen Zwecke geeignetes Stimulationsverfahren ist in Fig. 9A schematisch dargestellt. In Fig. 9A sind in den oberen vier Zeilen untereinander vier Sinusschwingungen mit den Frequenzen f₁, f₂, f₃ bzw. f₄ gegen die Zeit t aufgetragen, d.h., jede Zeile entspricht einem der Kanäle aus Fig. 6. Aus den dargestellten Sinusschwingungen werden akustischen Reize 51 gebildet. Zur Erzeugung von pulsförmigen Sinusschwingungen sind die vier Sinusschwingungen mit Rechteckfunktionen multipliziert worden. Jeder Sinusschwingungspuls wiederholt sich periodisch mit einer Frequenz fₛₜᵢₘ. Die Frequenz fₛₜᵢₘ = 1/Tₛₜᵢₘ kann im Bereich von 1 bis 30 Hz und insbesondere im Bereich von 1 bis 20 Hz liegen, kann aber auch kleinere oder größere Werte annehmen. Derartige Sequenzen von pulsförmigen Sinusschwingungen sind, wenn sie als akustische Reize 51 appliziert werden, geeignet, die neuronale Phase der jeweils stimulierten krankhaften Neuronen-Subpopulation 28 bis 31 zurückzusetzen. Der Phasenreset ergibt sich dabei nicht notwendigerweise bereits nach einem oder wenigen Pulsen, sondern es können eine gewisse Anzahl der in Fig. 9A gezeigten Sinusschwingungspulse 51 erforderlich sein, um die neuronale Phase der jeweiligen Subpopulation 28 bis 31 zurückzusetzen.

Die Frequenz fₛₜᵢₘ kann beispielsweise im Bereich der mittleren Frequenz der krankhaft rhythmischen Aktivität des Ziel-Netzwerks liegen. Bei neurologischen und psychiatrischen Erkrankungen liegt die mittlere Frequenz typischerweise im Bereich von 1 bis 30 Hz, kann aber auch außerhalb dieses Bereichs liegen. Beim Tinnitus findet sich z.B. im Frequenzbereich von 1,5 bis 4 Hz übermäßig synchrone neuronale Aktivität. Hierbei ist zu beachten, dass die Frequenz, mit welcher die krankhaften Neuronen synchron feuern, üblicherweise nicht konstant ist, sondern durchaus Variationen aufweisen kann und darüber hinaus bei jedem Patienten individuelle Abweichungen zeigt.

Zur Ermittlung der Frequenz fₛₜᵢₘ kann beispielsweise die mittlere Peakfrequenz der krankhaften rhythmischen Aktivität des Patienten mittels EEG- oder MEG-Messurigen bestimmt werden. Diese Peakfrequenz kann dann als Stimulationsfrequenz fₛₜᵢₘ verwendet werden oder auch variiert werden, beispielsweise in einem Bereich von fₛₜᵢₘ - 3 Hz bis fstim + 3 Hz.

Die Dauer eines Sinusschwingungspulses 51, d.h. die Zeitspanne, in dem in der vorliegenden Ausgestaltung die Rechteckfunktion den Wert 1 annimmt, kann beispielsweise Tₛₜᵢₘ/2 betragen. In diesem Fall sind die Zeitspanne, während der die jeweilige Frequenz zur Stimulation beiträgt, und die nachfolgende Stimulationspause gleich lang. Es ist aber auch möglich andere Stimulationsdauern zu wählen, beispielsweise im Bereich von Tₛₜᵢₘ/2 - Tₛₜᵢₘ/10 bis Tₛₜᵢₘ/2 + Tₛₜᵢₘ/ 10. Die Stimulationsdauern können beispielsweise experimentell bestimmt werden.

Gemäß der in Fig. 9A gezeigten Ausgestaltung erfolgt die Verabreichung der einzelnen Frequenzen f₁ bis f₄ mit einer zeitlichen Verzögerung zwischen den einzelnen Frequenzen f₁ bis f₄. Beispielsweise kann der Beginn zeitlich aufeinander folgender und unterschiedliche Frequenzen aufweisender Pulse um eine Zeit τ verschoben sein.

Im Fall von N Frequenzen, die zur Stimulation eingesetzt werden, kann die zeitliche Verzögerung τ zwischen jeweils zwei aufeinander folgenden Pulsen beispielsweise im Bereich eines N-tels der Periode Tₛₜᵢₘ = 1/fₛₜᵢₘ liegen. In dem in Fig. 9A gezeigten Ausführungsbeispiel (N = 4) beträgt die zeitliche Verzögerung τ dementsprechend Tₛₜᵢₘ/4. Von der Vorgabe, dass die zeitliche Verzögerung τ zwischen jeweils zwei aufeinander folgenden Sinusschwingungspulsen Tₛₜᵢₘ/N beträgt, kann bis zu einem gewissen Grad abgewichen werden. Beispielsweise kann von dem Wert Tₛₜᵢₘ/N für die zeitliche Verzögerung τ um bis zu ± 5%, ± 10% oder ± 20% abgewichen werden. Bei einer derartigen Abweichung wurden noch Stimulationserfolge erzielt, d.h., es konnte noch ein desynchronisierender Effekt beobachtet werden.

Aus den periodischen Sinusschwingungspulsen 51 mit den Frequenzen f₁ bis f₄ wird durch Superposition der akustische Reiz gebildet. Die einzelnen Sinusschwingungspulse 51 können dabei beispielsweise linear oder nichtlinear miteinander kombiniert werden. Dies bedeutet, dass die Sinusschwingungen der einzelnen Frequenzen f₁ bis f₄ nicht notwendigerweise mit den gleichen Amplituden zu dem akustischen Reiz kombiniert werden müssen. In der untersten Zeile von Fig. 9A ist beispielhaft das Frequenzspektrum des akustischen Reizes zu vier verschiedenen Zeitpunkten t₁, t₂, t₃ und t₄ dargestellt. Die dort gezeigten Frequenzspektren, insbesondere die Höhe und Form der Frequenzpeaks, sind lediglich beispielhaft zu verstehen und können auch völlig unterschiedliche Formen aufweisen. Im Einzelnen lassen sich den dargestellten Frequenzspektren die folgenden Aussagen entnehmen: Zum Zeitpunkt t₁ tritt lediglich die Frequenz f₁ in dem akustischen Reiz auf. Zum Zeitpunkt t₂ sind dies die Frequenzen f₃ sowie f₄, zum Zeitpunkt t₃ die Frequenzen f₂ bis f₄ und zum Zeitpunkt t₄ die Frequenzen f₂ sowie f₃.

Gemäß einer alternativen Ausgestaltung werden statt der Frequenzen f₁ bis f₄ vier Frequenzgemische mit den Grenzen f_{j,unten} und f_{j,oben} (j = 1, 2, 3, 4) verwendet. In einem Frequenzgemisch j kann eine beliebige Anzahl von Frequenzen im Bereich von f_{j,unten} bis f_{j,oben} vorliegen. Eine weitere Alternative stellen die in Fig. 8B gezeigten Tonpakete mit den Frequenzverteilungen v₁ bis v₄ dar.

Weitere Variationen der akustischen CR-Stimulation sind in Fig. 9B gezeigt. Da Einhüllende mit scharfen Kanten wie die in den Fig. 8A und 9A gezeigten Rechteckfunktionen zu Klickgeräuschen führen, wird in der Praxis ein glatterer Verlauf gewählt. Als Beispiel dafür ist in Fig. 9B eine CR-Stimulation mit Sinusschwingungspulsen 51 gezeigt, deren Einhüllende eine Cosinus-Halbwelle ist.

Des Weiteren zeigt Fig. 9B eine Abweichung von dem streng periodischen Stimulationsmuster aus Fig. 9A. Die Reihenfolge, in der die Reize 51 appliziert werden, ist in Fig. 9B pro Zyklus randomisiert worden. Außerdem ist eine Pause vorgesehen, während der keine Stimulation erfolgt. Derartige Pausen können beliebig lang gewählt werden und insbesondere ein ganzzahliges Vielfaches der Zyklusdauer betragen.

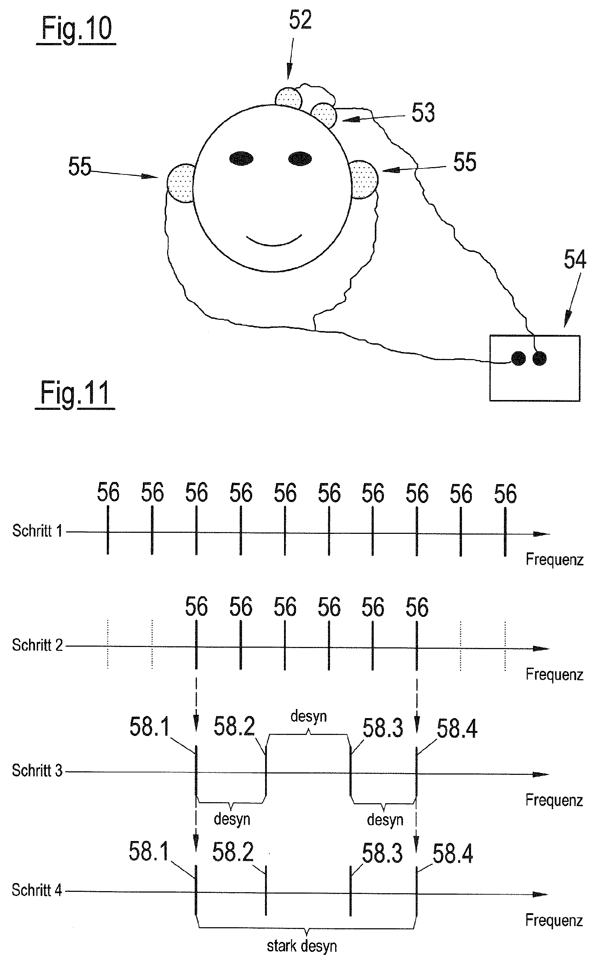

Fig. 10 zeigt schematisch eine Vorrichtung zur EEG-basierten Eichung der CR-Töne für die Behandlung von neurologischen und psychiatrischen Erkrankungen, z.B. Tinnitus, ADHS, Zwangserkrankungen. Nicht-invasiv fixierte EEG-Elektroden 52, 53, die über ein Kabel verbunden sind, dienen als Messeinheit und messen die EEG-Reizantworten, welche über ein Kabel an die zentrale Steuer- und Analyseeinheit 54 weitergeleitet werden. Akustische Testreize werden dem Patienten über Ohr- oder Kopfhörer 55 verabreicht. Die hierzu verwendeten Steuersignale werden von der Steuer- und Analyseeinheit 54 generiert und für die Datenanalyse der EEG-Reizantworten verwendet.

Im Folgenden wird die oben bereits allgemein beschriebene Eichung zur Bestimmung der optimalen Reizparameter in Bezug auf die akustische CR-Neuromodulation erläutert. Ein Flussdiagramm zur Veranschaulichung des Ablaufs der Eichung für die akustische CR-Neuromodulation ist in Fig. 11 dargestellt.

Im ersten Schritt werden mit Hilfe des Ohr- oder Kopfhörers 55 (oder allgemein der Stimulationseinheit) erste Reize 56 erzeugt und dem Patienten verabreicht. Im Fall einer Tinnitus-Behandlung können als erste Reize 56 beispielsweise Töne in einem ersten Frequenzintervall um die dominante Tinnitusfrequenz des Patienten (mit tonalem Tinnitus) als Startauswahl gewählt werden. Das erste Frequenzintervall kann gemäß der dem Fachmann bekannten physiologischen Abbildungscharakteristika (z.B. in erster Näherung logarithmische tonotope Karte im primären auditorischen Cortex) so mit den ersten Reizen 56 abgedeckt werden, dass diese die jeweilige kortikale Repräsentation in erster Näherung äquidistant abdecken, d.h., die zugehörigen Zielorte im Gehirn, die durch die ersten Reize 56 stimuliert werden, sollen in erster Näherung gleiche räumliche Entfernungen voneinander haben, also äquidistant sein.

Die ersten Reize 56 werden von der Steuer- und Analyseeinheit 54 darauf getestet, ob sie die Phase der pathologischen, synchronisierten und oszillatorischen Hirnaktivität zurücksetzen können. Dabei werden aus der Vorauswahl an ersten Reizen 56 diejenigen ersten Reize 56 selektiert, welche die Phase der pathologischen, synchronisierten und oszillatorischen Hirnaktivität zurückzusetzen vermögen. Fig. 11 zeigt beispielhaft die dabei ausgewählten ersten Reize 56 im Schritt 2. Die nicht wirksamen, d.h. nicht phasenrücksetzenden und dementsprechend verworfenen ersten Reize 56 sind im Schritt 2 gestrichelt dargestellt.

Sofern alle ersten Reize 56 aus der Startauswahl, d.h. alle im Schritt 1 der Fig. 11 gezeigten ersten Reize 56, die Phase der pathologischen, synchronisierten und oszillatorischen Hirnaktivität zurückzusetzen vermögen, werden diesen ersten Reize 56 noch zweite Reize ("Randreize") hinzugefügt (in Fig. 11 nicht dargestellt). Die zweiten Reize sind Töne, die außerhalb des ersten Frequenzintervalls liegen. Die zweiten Reize liegen innerhalb eines zweiten Frequenzintervalls, welches das erste Frequenzintervall umfasst. Auch unter den zweiten Reizen werden diejenigen Reize ausgewählt, die die Phase der pathologischen, synchronisierten und oszillatorischen Hirnaktivität zurücksetzen können.

Nachdem im zweiten Schritt die wirksamen, d.h. phasenrücksetzenden Töne selektiert worden sind, ermittelt die Steuer- und Analyseeinheit 54 im dritten Schritt diejenigen dritten Reize 58, die das zugehörige Hirnareal möglichst äquidistant reizen. Dazu wird folgendermaßen vorgegangen. Für den Fall, dass im zweiten Schritt keine zweiten Reize ausgewählt wurden, werden verteilt auf den gesamten wirksamen Frequenzbereich neben den außen gelegenen ersten Reizen 56 (d.h. den höchsten und tiefsten phasenrücksetzenden Tönen) gemäß dem Fachmann bekannter physiologischer Skalierung (z.B. logarithmische tonotope Skala) noch wenige weitere Reize ausgewählt, deren Frequenz zwischen den beiden außen gelegenen wirksamen ersten Reizen 56 liegt. Diese Reize bilden die dritten Reize 58. In Fig. 10 sind beispielhaft vier dritte Reize 58.1, 58.2, 58.3 und 58.4 dargestellt. Vorzugsweise sind die Zielorte der dritten Reize 58.1 bis 58.4 im Gehirn oder Rückenmark des Patienten näherungsweise äquidistant. Die Anzahl der dritten Reize 58.1 bis 58.4 ist typischerweise kleiner als die Anzahl der im zweiten Schritt ausgewählten ersten Reize 56.

Sofern im zweiten Schritt zweite Reize ausgewählt wurden, werden die im wirksamen (zweiten) Frequenzbereich außen gelegenen zweiten Reize als "Randreize" der dritten Reize 58 gewählt (entsprechend den Reizen 58.1 und 58.4 aus Fig. 11). Zwischen diesen äußeren zweiten Reizen werden noch weitere dritte Reize verteilt (entsprechend den Reizen 58.2 und 58.3 aus Fig. 11). Auch hier ist die Anzahl der dritten Reize vorzugsweise kleiner als die Anzahl der wirksamen ersten und zweiten Reize.

Mit den dritten Reize 58.1 bis 58.4 wird jeweils paarweise eine CR-Stimulation durchgeführt, wie sie in Fig. 9A gezeigt ist (in diesem Fall mit nur zwei Kanälen). Zunächst wird das Reizpaar 58.1/58.2 getestet. Sofern dieser Test einen Amplitudenanstieg der pathologischen Oszillation (also eine Verstärkung der Synchronisation der das Signal generierenden pathologisch synchronisierten Neuronenpopulation) ergeben sollte, wird der Reiz 58.2 solange zu höheren Frequenzen verschoben, bis die CR-Stimulation mit dem Reizpaar 58.1/58.2 keinen Amplitudenanstieg der pathologischen Oszillation mehr zeigt. Anschließend wird das Reizpaar 58.2/58.3 auf die gleiche Art getestet. Falls erforderlich wird hier der Reiz 58.3 zu höheren Frequenzen hin verschoben, bis bei der CR-Stimulation die Amplitude der pathologischen Oszillation nicht mehr wächst. Danach wird das Reizpaar 58.3/58.4 in gleicher Weise ausgetestet. Dabei kann es vorkommen, dass der äußerste Reiz 58.4 aus dem zweiten Frequenzintervall hinauswandert. In diesem Fall kann gegebenenfalls der Reiz 58.4 oder ein anderer Reiz, z.B. der Reiz 58.3, verworfen werden. Im letzteren Fall würde der Test mit dem Reizpaar 58.2/58.4 nochmals durchgeführt werden.

Im vierten Schritt prüft die Steuer- und Analyseeinheit 54, ob die CR-Stimulation bei Verwendung aller im dritten Schritt bestimmten dritten Reize 58.1 bis 58.4 die krankhaft synchrone und oszillatorische neuronale Aktivität der stimulierten Neuronen unterdrückt und insbesondere desynchronisierend wirkt. Dazu wird eine CR-Stimulation durchgeführt, wie sie in Fig. 9A gezeigt ist. Eine solche CR-Stimulation mit allen selektierten dritten Reizen 58.1 bis 58.4 sollte zu einer Abnahme der Amplitude des pathologischen Signals führen, was einer CR-induzierten Desynchronisation der zugrundeliegenden, krankhaft synchronisierten Neuronenpopulation entspricht.

### Stimulationseinheiten zur Erzeugung optischer Reize:

Im Folgenden werden Ausgestaltungen der nicht-invasiven Stimulationseinheit 11 zur Erzeugung optischer Reize 22 beschrieben. Derartige Stimulationseinheiten lassen sich auch der deutschen Patentanmeldung Nr. 10 2008 012 669.1 mit dem Titel "Vorrichtung und Verfahren zur visuellen Stimulation" entnehmen, die am 5. März 2008 beim Deutschen Patent- und Markenamt hinterlegt worden ist. Der gesamte Offenbarungsgehalt der deutschen Patentanmeldung Nr. 10 2008 012 669.1 wird hiermit in die Offenbarung der vorliegenden Anmeldung aufgenommen.

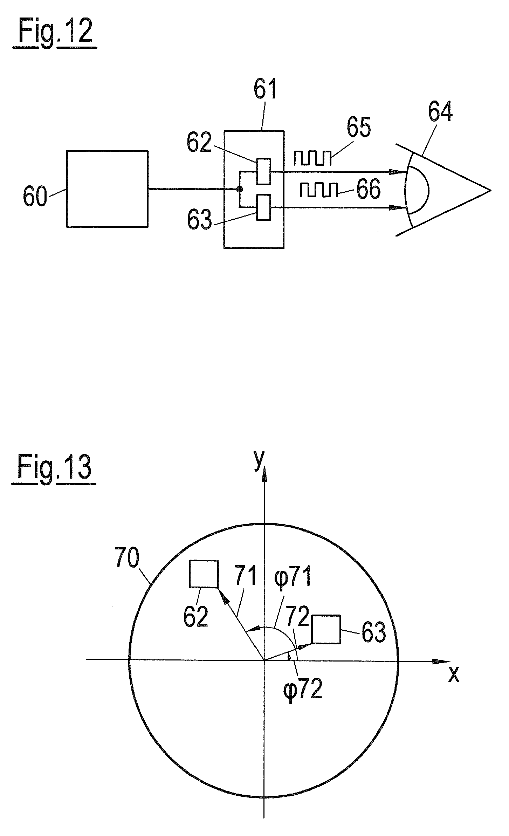

Fig. 12 zeigt schematisch eine Steuer- und Analyseeinheit 60 und eine von der Steuer- und Analyseeinheit 60 angesteuerte Stimulationseinheit 61 (die Messeinheit ist in Fig. 12 nicht dargestellt). Die Stimulationseinheit 61 beinhaltet eine Mehrzahl von Stimulationselementen zur Erzeugung von optischen Reizen. In dem vorliegenden Ausführungsbeispiel weist die Stimulationseinheit 61 zwei Stimulationselemente 62 und 63 auf, die von der Steuer- und Analyseeinheit 60 angesteuert werden. In Fig. 12 ist ferner ein Auge 64 eines Patienten dargestellt.

Während des Betriebs der Stimulationseinheit 61 erzeugen die Stimulationselemente 62 und 63 optische Reize 65 bzw. 66, die vom Patienten über ein oder beide Augen 64 aufgenommen werden und über die Sehnerven an Neuronenpopulationen im Gehirn weitergeleitet werden.

Den optischen Reizen 65, 66 kann eine Leuchtstärken- bzw. Helligkeitsvariation (bzw. Variation der Lichtintensität oder Lichtstärke) zugrunde liegen, beispielsweise können sie als Pulse oder als Sequenzen von Pulsen mit variierter Leuchtstärke bzw. Helligkeit appliziert werden. Die optischen Reize 65, 66 können je nach Ausgestaltung der Stimulationseinheit 61 als Leuchtstärkenmodulation natürlicher optischer Reize, z.B. mittels einer homogenen oder segmentierten Transmissionsbrille, als zusätzlich zu einem natürlichen optischen Reiz auftretender, modulierter optischer Reiz, z.B. mittels einer partiell durchsichtigen Lichtbrille, oder als künstlicher optischer Helligkeitsreiz, z.B. mittels einer undurchsichtigen Lichtbrille, verabreicht werden. Falls der Patient die optischen Reize 65, 66 über beide Augen 64 aufnimmt, können die jeweiligen optischen Reize 65, 66 beider Augen 64 korreliert bzw. koordiniert werden.

Die von den Stimulationselementen 62, 63 erzeugten optischen Reize 65, 66 können derart ausgestaltet sein, dass sie, wenn sie von der Retina aufgenommen werden und über den Sehnerv zu einer Neuronenpopulation mit einer krankhaft synchronen und oszillatorischen Aktivität geleitet werden, in der Neuronenpopulation ein Zurücksetzen der Phase der neuronalen Aktivität der stimulierten Neuronen bewirken.

In Fig. 13 ist schematisch das Gesichtsfeld 70 eines Patienten dargestellt. Als Gesichtsfeld wird der Raum bezeichnet, der mit einem Auge ohne Augenbewegungen überblickt wird. In Fig. 13 ist das Gesichtsfeld 70 zur Vereinfachung kreisförmig dargestellt. Typischerweise hat das Gesichtsfeld eine eher gewölbte ovale Form. Die genaue Größe und Form des Gesichtsfelds unterliegt dabei individuellen Schwankungen und ist zudem altersabhängig.

Punkte im Gesichtsfeld 70 lassen sich beispielsweise mit Hilfe ihrer Polarkoordinaten beschreiben. In Fig. 13 sind die räumlichen Lagen der Stimulationselemente 62 und 63 im Gesichtsfeld 70 beispielhaft dargestellt. Zur Veranschaulichung ist jeweils ein Eckpunkt der Stimulationselemente 62 und 63 mit einem Vektor 71 bzw. 72 gekennzeichnet. Die Vektoren 71 und 72 lassen sich im Polarkoordinatensystem über ihren Betrag und den Winkel ϕ₇₁ bzw. ϕ₇₂, den sie mit der x-Achse einschließen beschreiben.

Unterschiedliche Stellen im Gesichtsfeld 70 werden über die Linse des Auges auf unterschiedliche Stellen der Retina abgebildet. Die unterschiedlichen Stellen der Retina sind wiederum über den Sehnerv mit unterschiedlichen Neuronen im Gehirn verbunden. Dies bedeutet, dass mit den an unterschiedlichen räumlichen Orten angeordneten Stimulationselementen 62 und 63 jeweils unterschiedliche Neuronen stimuliert werden können. Folglich können die Stimulationselemente 62 und 63 sowie evtl. weitere Stimulationselemente räumlich so im Gesichtsfeld 70 des Patienten angeordnet sein, dass die von der Retina aufgenommenen optischen Reize an unterschiedliche Zielgebiete im Gehirn weitergeleitet werden. Es können demnach unterschiedliche Subpopulationen einer krankhaften Neuronenpopulation mit den Stimulationselementen 62 und 63 gezielt stimuliert werden, und es kann ein zeitversetztes Zurücksetzen der Phasen dieser Subpopulation durchgeführt werden.

Die Zuordnung der Bereiche des Gesichtsfelds zu entsprechenden Bereichen des Gehirns ist beispielsweise in dem Artikel "Visual Field Maps in Human Cortex" von B. A. Wandell, S. O. Dumoulin und A. A. Brewer, erschienen in Neuron 56, Oktober 2007, Seiten 366 bis 383, beschrieben.

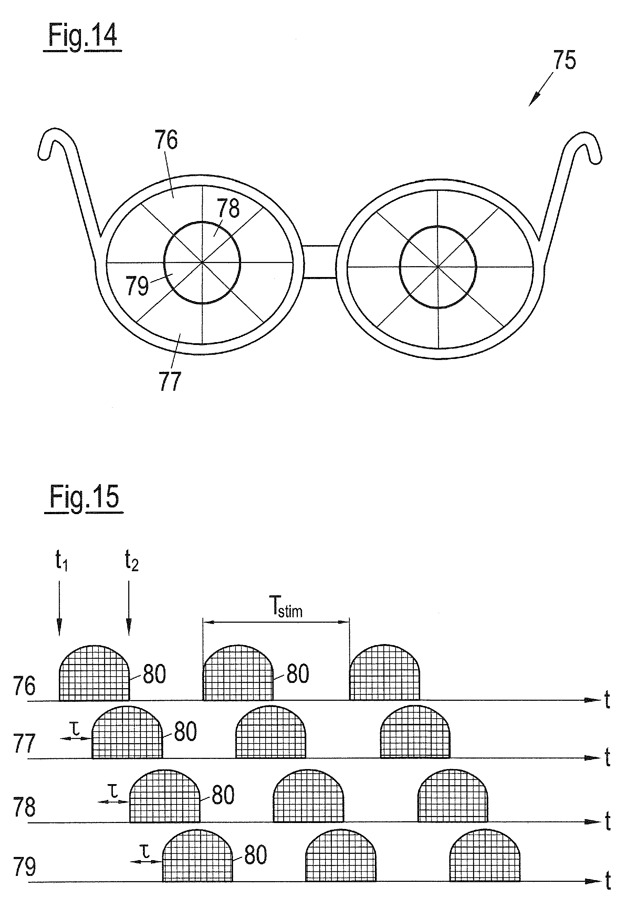

In Fig. 14 ist schematisch eine Ausgestaltung der Stimulationseinheit 61 als Transmissionsbrille 75 mit segmentierten Transmissionsgläsern dargestellt. Die Transmissionsgläser sind jeweils in unterschiedliche Segmente unterteilt, deren Transmission getrennt gesteuert werden kann. Die Segmentierung kann beispielsweise radial und/oder zirkular sein (beides ist in Fig. 14 gezeigt). Die in Fig. 14 gezeigte Transmissionsbrille 75 ist lediglich beispielhaft zu verstehen. Die Anzahl der Segmente sowie die geometrischen Formen der einzelnen Segmente können anders gewählt werden.

Die Segmente der Transmissionsbrille 75 entsprechen den in Fig. 12 gezeigten Stimulationselementen. In Fig. 14 sind beispielhaft vier der Segmente mit den Bezugszeichen 76, 77, 78 und 79 gekennzeichnet.

Anhand der Segmente 76 bis 79 soll nachfolgend beispielhaft erläutert werden, wie mittels einer CR-Neuromodulation, also durch zeitversetztes Zurücksetzen der Phasen von Subpopulationen einer krankhaft synchronen und oszillatorischen Neuronenpopulation, eine Desynchronisation der gesamten Neuronenpopulation erzielt werden kann. Die Segmente 76 bis 79 sind so ausgewählt worden, dass die von ihnen erzeugten optischen Reize jeweils vorzugsweise von einem bestimmten Teil der Netzhaut des Patienten aufgenommen werden, von wo aus die Reize zu bestimmten Bereichen des Gehirns weitergeleitet werden, so dass die oben beschriebene Aufspaltung einer krankhaften Neuronenpopulation in Subpopulationen ermöglicht wird (vgl. z.B. Neuronenpopulation 27 mit Subpopulationen 28 bis 31 in Fig. 1). Damit Subpopulationen mit unterschiedlichen Phasen gebildet werden, können die optischen Reize von den Segmenten 76 bis 79 beispielsweise zeitversetzt erzeugt werden. Gleichbedeutend mit dem zeitversetzten Erzeugen der Reize ist ein phasenversetztes Erzeugen der Reize, welches im Ergebnis ebenfalls zu einem zeitversetzten Zurücksetzen der Phasen der unterschiedlichen Subpopulationen führt.

Ein für die oben beschriebenen Zwecke geeignetes Stimulationsverfahren, das beispielsweise mit der Transmissionsbrillen 75 durchgeführt werden kann, ist in Fig. 15 schematisch dargestellt. In Fig. 15 sind untereinander die mittels der Segmente 76 bis 79 applizierten optischen Reize 80 gegen die Zeit t aufgetragen (die Segmente 76 bis 79 entsprechen den vier Kanälen aus Fig. 6). Bei der in Fig. 15 gezeigten Ausführungsform wird davon ausgegangen, dass nur die Segmente 76 bis 79 der Transmissionsbrille 75 optische Reize 80 erzeugen, d.h., nur die Transmission dieser Segmente wird von der Steuer- und Analyseeinheit 60 moduliert. Selbstverständlich ist dies nur beispielhaft zu verstehen. Bei alternativen Ausgestaltungen können anstelle der Segmente 76 bis 79 andere Segmente zum Generieren der optischen Reize herangezogen werden. Es ist möglich, wie in Fig. 15 nur eine Auswahl der Segmente der Transmissionsbrille 75 zur Stimulation zu verwenden oder auch sämtliche Segmente.

Bei dem in Fig. 15 dargestellten Verfahren appliziert jedes der Segmente 76 bis 79 periodisch den optischen Reiz 80. Pro Segment 76 bis 79 wird der Reiz 80 in dem vorliegenden Beispiel dreimal appliziert. Alternativ könnte der Reiz 145 pro Sequenz beispielsweise auch ein- bis zwanzigmal wiederholt werden. Die Frequenz fₛₜᵢₘ = 1/Tₛₜᵢₘ, mit welcher die Reize 80 pro Segment 76 bis 79 wiederholt werden, kann im Bereich von 1 bis 30 Hz und insbesondere im Bereich von 1 bis 20 Hz liegen, kann aber auch kleinere oder größere Werte annehmen. Derartige Sequenzen von optischen Reizen sind geeignet, die neuronale Phase einer stimulierten krankhaften Subpopulation von Neuronen zurückzusetzen. Die Frequenz fₛₜᵢₘ kann beispielsweise im Bereich der mittleren Frequenz der krankhaft rhythmischen Aktivität des Ziel-Netzwerks liegen, wie bereits oben erläutert wurde.

Die Struktur eines einzelnen optischen Reizes 80 soll nachfolgend anhand des ersten von dem Segment 76 generierten Reizes 80 erläutert werden. Hier wird zum Zeitpunkt t₁ das Segment 76 von der Steuer- und Analyseeinheit 60 derart angesteuert, dass die Transmission, d.h. die Lichtdurchlässigkeit des Segments 76 verringert wird. Zum Zeitpunkt t₂ schaltet die Steuer- und Analyseeinheit 60 die Transmission des Segments 76 auf den maximalen Wert. In anderen Worten bedeutet dies, dass das Segment 76 weniger transparent wird, wenn stimuliert wird. Dementsprechend nimmt der Patient eine verringerte Helligkeit des Umgebungslichts im Bereich des Segments 76 während der Stimulation wahr.

Die Einzelpulse 80 haben vorzugsweise keine Rechteckform sondern weniger scharfe Flanken. Es können aber auch - z.B. je nach Grunderkrankung des Patienten sowie individueller psychophysischer Beschaffenheit, z.B. Blendempfindlichkeit - anders ausgestaltete Reize, z.B. sinusförmige optische Reize, verwendet werden.

Alternativ ist es auch möglich, die Transmission des Segments 76 zum Zeitpunkt t₁ zu erhöhen und zum Zeitpunkt t₂ minimal zu schalten, so dass das Segment 76 während der Stimulation stärker transparent wird.

Grundsätzlich ist es denkbar, als maximale Transmission 100% zu wählen, d.h., in diesem Fall wird das Umgebungslicht durch das jeweilige Segment überhaupt nicht abgeschwächt. Eine derart hohe Transmission lässt sich jedoch aufgrund technischer Beschränkungen häufig nicht erreichen, so dass kleinere Transmissionswerte für die maximale Transmission im Bereich von 60% bis 100% gewählt werden können. Die minimale Transmission kann einen Wert in dem Bereich von 0% bis 30% annehmen. Es können aber auch noch Stimulationserfolge mit Transmissionswerten, die außerhalb der angegebenen Bereiche liegen, erzielt werden.

Die Dauer eines optischen Reizes 80, d.h. die Zeitspanne zwischen den Zeitpunkten t₁ und t₂, kann beispielsweise Tₛₜᵢₘ/2 betragen. In diesem Fall sind die Zeitspanne, während der stimuliert wird, und die nachfolgende Stimulationspause gleich lang (falls nur über zwei Brillensegmente stimuliert wird). Es ist aber auch möglich andere Stimulationsdauern zu wählen, beispielsweise im Bereich von Tₛₜᵢₘ/2 - Tₛₜᵢₘ/10 bis Tₛₜᵢₘ/2 + Tₛₜᵢₘ/ 10. Auch andere Stimulationsdauern sind möglich und können beispielsweise experimentell bestimmt werden.

Gemäß der in Fig. 15 gezeigten Ausgestaltung erfolgt die Verabreichung der optischen Reize 80 über die einzelnen Segmente 76 bis 79 der Transmissionsbrille 11 mit einer zeitlichen Verzögerung zwischen den einzelnen Segmenten 76 bis 79. Beispielsweise kann der Beginn zeitlich aufeinander folgender und von unterschiedlichen Segmenten 76 bis 79 applizierten Reizen 80 um eine Zeit τ verschoben sein.

Im Fall von N Stimulationselementen bzw. Segmenten, die zur Stimulation eingesetzt werden, kann die zeitliche Verzögerung τ zwischen jeweils zwei aufeinander folgenden Reizen 80 beispielsweise im Bereich eines N-tels der Periode Tₛₜᵢₘ = 1/fₛₜᵢₘ liegen. In dem in Fig. 15 gezeigten Ausführungsbeispiel (N = 4) beträgt die zeitliche Verzögerung τ dementsprechend Tₛₜᵢₘ/4. Von der Vorgabe, dass die zeitliche Verzögerung τ zwischen jeweils zwei aufeinander folgenden Reizen 80 Tₛₜᵢₘ/N beträgt, kann bis zu einem gewissen Grad abgewichen werden. Beispielsweise kann von dem Wert Tₛₜᵢₘ/N für die zeitliche Verzögerung τ um bis zu ± 5%, ± 10% oder ± 20% abgewichen werden. Bei einer derartigen Abweichung wurden noch Stimulationserfolge erzielt, d.h., es konnte noch ein desynchronisierender Effekt beobachtet werden.

Zur Bestimmung der optimalen Reizparameter für eine optische CR-Neuromodulation kann die bereits oben erläuterte Eichprozedur durchgeführt werden.

Im ersten Schritt der Eichprozedur werden mittels der Transmissionsbrille 75 oder einer anderen optischen Stimulationseinheit erste optische Reize erzeugt, die in einem ersten Gesichtsfeldbereich liegen. Der erste Gesichtsfeldbereich soll gemäß der dem Fachmann bekannten physiologischen Abbildungscharakteristika so mit den ersten Reizen abgedeckt werden, dass die zugehörigen Zielorte im Gehirn, die durch die ersten optischen Reize stimuliert werden, in erster Näherung gleiche räumliche Entfernungen voneinander haben, also äquidistant sind.

Die ersten Reize werden anschließend von der Steuer- und Analyseeinheit 60 darauf getestet, ob sie die Phase der pathologischen, synchronisierten und oszillatorischen Hirnaktivität zurücksetzen können. Dabei werden im zweiten Schritt aus der Vorauswahl an ersten Reizen diejenigen ersten Reize selektiert, welche die Phase der pathologischen, synchronisierten und oszillatorischen Hirnaktivität zurückzusetzen vermögen.

Sofern alle ersten Reize aus der Startauswahl die Phase der pathologischen, synchronisierten und oszillatorischen Hirnaktivität zurücksetzen können, werden diesen ersten Reize noch zweite Reize ("Randreize") hinzugefügt. Die zweiten Reize liegen außerhalb des ersten Gesichtsfeldbereichs aber innerhalb eines zweiten Gesichtsfeldbereichs, welcher den ersten Gesichtsfeldbereich umfasst. Auch unter den zweiten Reizen werden diejenigen Reize ausgewählt, die die Phase der pathologischen, synchronisierten und oszillatorischen Hirnaktivität zurücksetzen können.

Nachdem im zweiten Schritt die wirksamen, d.h. phasenrücksetzenden Reize selektiert worden sind, ermittelt die Steuer- und Analyseeinheit 60 im dritten Schritt diejenigen dritten Reize, die das zugehörige Hirnareal bei Geradeaus-Blick möglichst äquidistant reizen. Für den Fall, dass im zweiten Schritt keine zweiten Reize ausgewählt wurden, werden verteilt auf den gesamten wirksamen Gesichtsfeldbereich neben den außen gelegenen ersten Reizen gemäß dem Fachmann bekannter physiologischer Skalierung (z.B. komplex logarithmische Abbildung der Retina auf den Cortex) noch wenige weitere Reize ausgewählt, die zwischen den beiden im wirksamen Gesichtsfeldbereich außen gelegenen ersten Reizen liegen. Diese Reize bilden die dritten Reize. Vorzugsweise sind die Zielorte der dritten Reize im Gehirn oder Rückenmark des Patienten näherungsweise äquidistant.

Sofern zweite Reize ausgewählt wurden, werden die im wirksamen (zweiten) Gesichtsfeldbereich außen gelegenen zweiten Reize als "Randreize" der dritten Reize gewählt. Zwischen diesen äußeren zweiten Reizen werden noch weitere dritte Reize verteilt.

Mit den dritten Reizen wird jeweils paarweise eine CR-Stimulation durchgeführt, wie sie in Fig. 15 gezeigt ist (in diesem Fall mit nur zwei Segmenten der Transmissionsbrille 75). Sofern dieser Test einen Amplitudenanstieg der pathologischen Oszillation ergeben sollte, wird der Abstand der beiden dritten Reize solange variiert (durch Verwendung anderer Segmente der Transmissionsbrille 75), bis die CR-Stimulation mit dem Reizpaar keinen Amplitudenanstieg der pathologischen Oszillation mehr zeigt. Dieser Test wird mit allen benachbarten dritten Reizen durchgeführt.

Im vierten Schritt prüft die Steuer- und Analyseeinheit 60, ob bei Verwendung aller im dritten Schritt bestimmten dritten Reize die zugehörige CR-Neuromodulation die krankhaft synchrone und oszillatorische neuronale Aktivität der stimulierten Neuronen unterdrückt und insbesondere desynchronisierend wirkt. Dazu wird eine CR-Stimulation durchgeführt, wie sie in Fig. 15 gezeigt ist. Eine solche CR-Stimulation mit allen selektierten dritten Reizen sollte zu einer Abnahme der Amplitude des pathologischen Signals führen, was einer CR-induzierten Desynchronisation der zugrundeliegenden, krankhaft synchronisierten Neuronenpopulation entspricht.

### Stimulationseinheiten zur Erzeugung taktiler, vibratorischer, thermischer und/oder elektrischer transkutaner Reize:

Im Folgenden werden Ausgestaltungen der nicht-invasiven Stimulationseinheit 11 zur Erzeugung taktiler, vibratorischer, thermischer und/oder elektrischer transkutaner Reize 22 beschrieben. Derartige Stimulationseinheiten lassen sich auch der deutschen Patentanmeldung Nr. 10 2010 000 390.5 mit dem Titel "Vorrichtung und Verfahren zur Behandlung eines Patienten mit Vibrations-, Tast- und/oder Thermoreizen" entnehmen, die am 11. Februar 2010 beim Deutschen Patent- und Markenamt hinterlegt worden ist. Der gesamte Offenbarungsgehalt der deutschen Patentanmeldung Nr. 10 2010 000 390.5 wird hiermit in die Offenbarung der vorliegenden Anmeldung aufgenommen.

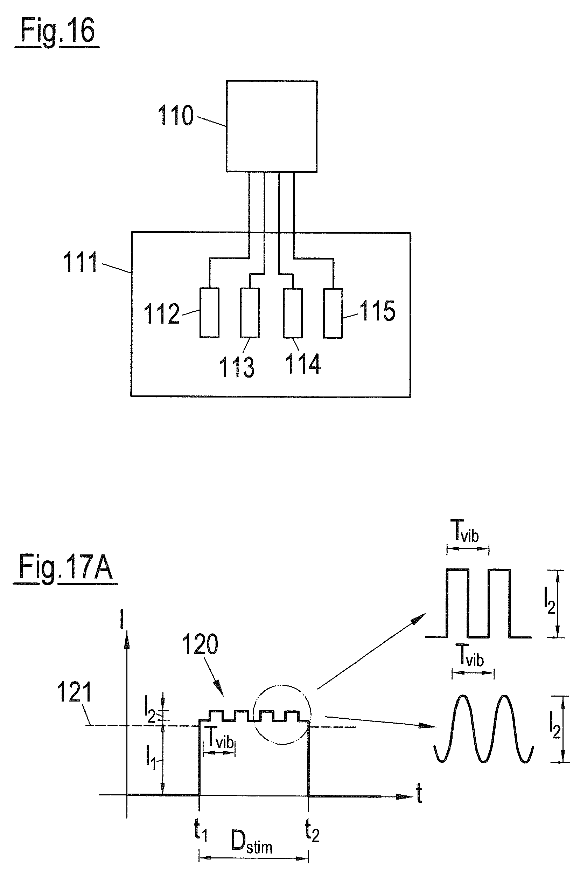

Fig. 16 zeigt schematisch eine Steuer- und Analyseeinheit 110 und eine von der Steuer- und Analyseeinheit 110 angesteuerte Stimulationseinheit 111 (die Messeinheit ist in Fig. 16 nicht dargestellt). Die Stimulationseinheit 111 beinhaltet eine Mehrzahl von Stimulationselementen zur Erzeugung von taktilen, vibratorischen, thermischen und/oder elektrischen transkutanen Reizen. In dem vorliegenden Ausführungsbeispiel weist die Stimulationseinheit 111 vier Stimulationselemente 112, 113, 114 und 115 auf, die von der Steuer- und Analyseeinheit 110 angesteuert werden. Die in Fig. 16 gezeigte Ausgestaltung ist lediglich beispielhaft zu verstehen. Alternativ zu dieser Ausgestaltung kann die Stimulationseinheit 111 eine beliebige Anzahl von Stimulationselementen enthalten.

Die Stimulationselemente 112 bis 115 sind derart ausgestaltet, dass sie auf die Haut des Patienten aufgesetzt werden können. Je nach Erkrankung bzw. betroffenen Körperpartien werden die Stimulationselemente 112 bis 115 in einer geeigneten Anordnung auf der Haut des Patienten befestigt, beispielsweise am Arm, am Bein, an der Hand und/oder am Fuß des Patienten. Taktile, vibratorische, thermische und elektrische transkutane Reize können je nach Krankheitsbild entweder einzeln oder in Kombination auf der Haut verabreicht werden.

Die Mehrzahl von Stimulationselementen 112 bis 115 ermöglicht es, unterschiedliche rezeptive Bereiche der Haut über die einzelnen Stimulationselemente 112 bis 115 zeitlich und räumlich koordiniert zu stimulieren. Die Stimulationselemente 112 bis 115 können so auf der Haut des Patienten angeordnet sein, dass die auf das Hautgewebe applizierten Reize über Nervenleitungen an unterschiedliche Zielgebiete, die z.B. im Gehirn und/ oder Rückenmark liegen, weitergeleitet werden. Folglich können verschiedene Zielgebiete im Gehirn und/oder Rückenmark während desselben Stimulationszeitraums mit eventuell unterschiedlichen und/oder zeitversetzten Reizen stimuliert werden.

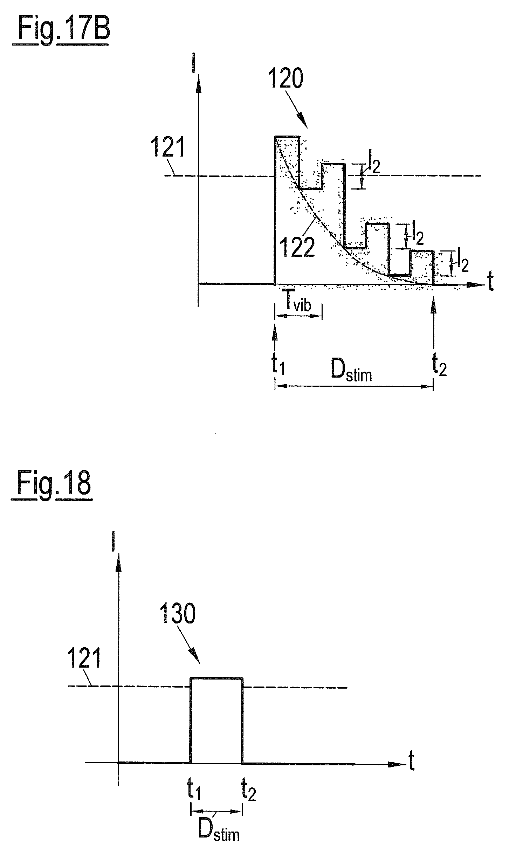

Verschiedene Ausgestaltungen einzelner vibratorischer Reize 120 sind in Fig. 17A und 17B dargestellt. Dort ist die Auslenkung 1 eines Stimulationselements gegen die Zeit t aufgetragen. In Fig. 17A wird das Stimulationselement zur Zeit t₁ aus seiner Ruheposition ausgelenkt und in die Haut des Patienten eingedrückt. Die Lage der Hautoberfläche ist durch eine gestrichelte Linie 121 dargestellt. Nachdem das Stimulationselement in Kontakt mit der Haut getreten ist, wird ein periodischer Vibrationsreiz mit einer Frequenz f_{vib} = 1/T_{vib} im Bereich von 5 bis 300 Hz appliziert (T_{vib} = Periodendauer des Vibrationsreizes). Bei einer Frequenz f_{vib} von 300 Hz kann das Stimulationselement eine Kraft von etwa 2 N ausüben. Die Dauer Dₛₜᵢₘ des Vibrationsreizes 120 kann im Bereich von 10 bis 500 ms liegen.

Zur Zeit t₂ wird das Stimulationselement wieder in seine Ruheposition verbracht, wo es keinen Kontakt zur Haut hat. Wie in Fig. 17A gezeigt kann der vibratorische Reiz 120 ein rechteckförmiger oder sinusförmiger Reiz sein, er kann aber auch andere Formen haben. Die in Fig. 17A gezeigte Auslenkung l₁ zur Eindrückung des Stimulationselements in die Haut kann im Bereich von 0,5 bis 3 mm liegen. Die Auslenkung l₂ des Stimulationselements während der Vibration kann zwischen 0,1 und 0,5 mm betragen.

Alternativ kann vorgesehen sein, dass das Stimulationselement stets in Kontakt mit der Haut des Patienten steht und ein rein vibratorischer Reiz während des Stimulationszeitraums Dₛₜᵢₘ appliziert wird.

Eine weitere Variante des vibratorischen Reizes 120 ist in Fig. 17B dargestellt. Im Unterschied zur in Fig. 17A gezeigten Ausgestaltung wird das Stimulationselement bereits während des Stimulationszeitraums Dₛₜᵢₘ wieder zurückgefahren, so dass die Vibrationen mit zunehmender Zeitdauer weniger in die Haut eindrücken und sich das Stimulationselement schließlich vollständig von der Haut löst. Beispielsweise kann das Zurückfahren des Stimulationselements entlang einer linearen oder nichtlinearen, z.B. exponentiellen, Kurve 122 erfolgen, welcher die Vibrationen f_{vib} des Stimulationselements überlagert sind. In dem in Fig. 17B gezeigten Beispiel reicht die abfallende Flanke eines jeden Pulses bis auf die Kurve 122 herunter. Der sich daran anschließende Puls hat eine fest vorgegebene Höhe l₂, d.h., die ansteigende Flanke eines jeden Pulses hat die Höhe l₂.

Eine Ausführungsform eines taktilen Reizes 130 ist in Fig. 18 gezeigt. Das Stimulationselement wird zur Zeit t₁ in die Haut des Patienten eingedrückt, verweilt dort für die Stimulationsdauer Dₛₜᵢₘ und wird zur Zeit t₂ wieder zurückgefahren. Die Stimulationsdauer Dₛₜᵢₘ liegt bei einem taktilen Reiz 130 im Bereich von 10 bis 500 ms.

Stimulationselemente zur Erzeugung taktiler und/oder vibratorischer Reize können beispielsweise als Stab oder Stempel ausgestaltet sein, mit dessen einem Ende die Haut des Patienten stimuliert wird. Das Ende des Stimulationselements, das in Berührung mit der Hautoberfläche kommt und letztlich die Reize erzeugt, kann beispielsweise im Wesentlichen die Form einer Halbkugel aufweisen oder eine noppenartige Oberfläche oder eine andere geeignete Form haben. Angetrieben wird das Stimulationselement von einem elektromechanischen Wandler (oder Aktor oder Aktuator), der elektrische Energie in eine Bewegung des Stimulationselements umsetzt. Als elektromechanische Wandler eignen sich beispielsweise Gleichstrommotoren, Schwingspulen (engl.: voice coil), piezoelektrische Wandler oder aus elektroaktiven Polymeren (EAP) aufgebaute Wandler, die beim Anlegen einer elektrischen Spannung ihre Form ändern.

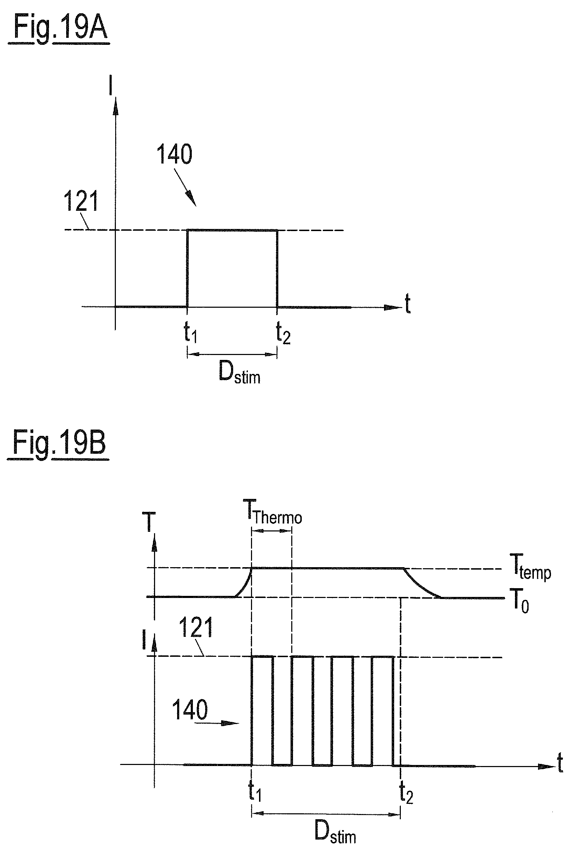

Verschiedene Ausführungsformen einzelner thermischer Reize 140 sind in Fig. 19A und 19B dargestellt. Bei beiden Ausgestaltungen wird ein Stimulationselement auf eine Temperatur Tₜₑₘₚ erhitzt oder gekühlt. Wie in Fig. 19B gezeigt ist, kann die Temperatur Tₜₑₘₚ erst kurz vor der Applikation des thermischen Reizes 140 erzeugt werden. In diesem Fall hat das Stimulationselement während der Stimulationspausen eine Temperatur T₀, welche z.B. der Raumtemperatur entspricht. Alternativ kann das Stimulationselement auf einer konstanten Temperatur Tₜₑₘₚ gehalten werden.

Bei der Ausgestaltung nach Fig. 19A wird das erhitzte oder gekühlte Stimulationselement zur Zeit t₁ auf die Haut des Patienten gebracht und verbleibt dort für die gesamte Stimulationsdauer Dₛₜᵢₘ. Im Unterschied dazu wird bei der Ausgestaltung nach Fig. 19B das Stimulationselement während der Stimulationsdauer Dₛₜᵢₘ periodisch mit einer Frequenz fₜₕₑᵣₘₒ zur Haut verbracht und wieder entfernt. Die Frequenz fₜₕₑᵣₘₒ = 1/Tₜₕₑᵣₘₒ kann im Bereich von 1 bis 10 Hz liegen (Tₜₕₑᵣₘₒ = Periodendauer des Thermoreizes).

Stimulationselemente, die durch Berührung der Hautoberfläche Thermoreize applizieren, können beispielsweise stabförmig ausgebildet sein und Heiz- und/oder Kühlelemente enthalten (z.B. in Form von Heizschleifen), welche die Stimulationselemente heizen oder kühlen. Für die Bewegung der Stimulationselemente können elektromechanische Wandler sorgen.

Bei einer weiteren Variante wird der thermische Reiz 140 berührungslos erzeugt. Hier wird die Stimulationstemperatur Tₜₑₘₚ durch elektromagnetische Strahlung, beispielsweise Infrarotlicht, erzeugt. Ferner wird die elektromagnetische Strahlung periodisch mit der Frequenz fₜₕₑᵣₘₒ = 1/Tₜₕₑᵣₘₒ variiert (z.B. durch An- und Ausschalten eines Infrarotstrahlers).

Bei thermischen Reizen 140 liegt die Stimulationsdauer Dₛₜᵢₘ im Bereich von 10 bis 500 ms. Die Temperatur Tₜₑₘₚ kann von 22 bis 42 °C betragen. Die Temperatur T₀ ist in der Regel die Körpertemperatur des Patienten. Die Frequenz fₜₕₑᵣₘₒ kann zwischen 1 und 10 Hz liegen, kann aber auch außerhalb dieses Bereichs liegen.

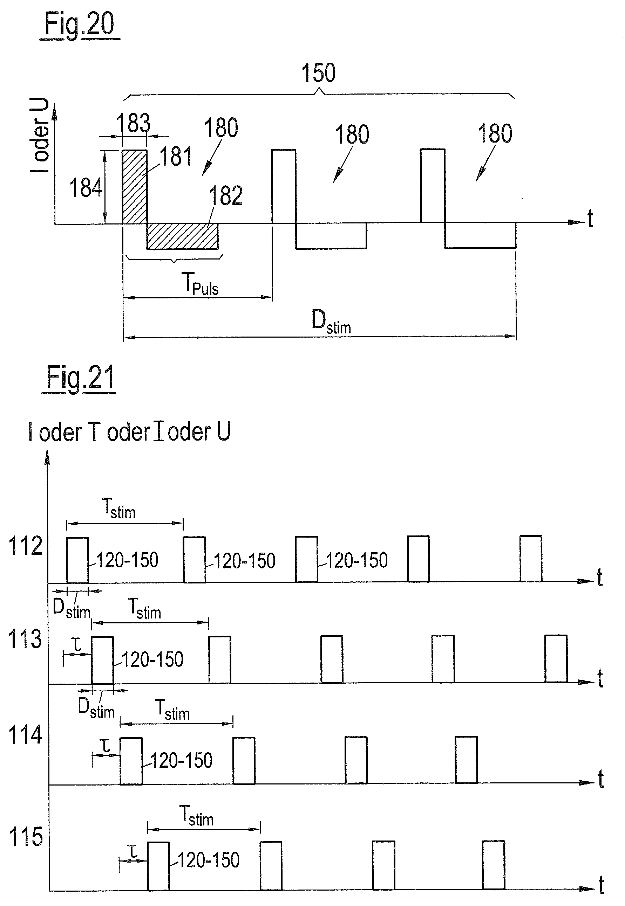

Fig. 20 zeigt einen elektrischen transkutanen Reiz 150, bei dem ein Strom- oder Spannungspulszug für die Dauer Dₛₜᵢₘ auf die Haut des Patienten appliziert wird. Der elektrische transkutane Reiz 150 kann von einer auf der Haut des Patienten befestigten Metallelektrode erzeugt werden.

Die elektrischen transkutanen Reize können z.B. ladungsbalanzierte Rechteck-Einzelpulse oder Pulszüge mit mehreren (z.B. 1 bis 100) ladungsbalanzierten Rechteck-Einzelpulsen sein. Der in Fig. 20 beispielhaft dargestellte Pulszug 150 besteht aus drei Einzelpulsen 180, die mit einer Frequenz f_{Puls} = 1/T_{Puls} im Bereich von 1 bis 150 Hz, insbesondere im Bereich von 60 bis 150 Hz, wiederholt werden. Die Einzelpulse 180 können strom- oder spannungskontrollierte Pulse sein, die sich aus einem anfänglichen Pulsanteil 181 und einem sich daran anschließenden, in entgegengesetzter Richtung fließenden Pulsanteil 182 zusammensetzen, wobei die Polarität der beiden Pulsanteile 181 und 182 gegenüber der in Fig. 20 gezeigten Polarität auch vertauscht werden kann. Die Dauer 183 des Pulsanteils 181 liegt im Bereich zwischen 1 µs und 450 µs. Die Amplitude 184 des Pulsanteils 181 liegt im Falle von stromkontrollierten Pulsen im Bereich zwischen 0 mA und 25 mA und im Fall von spannungskontrollierten Pulsen im Bereich von 0 bis 20 V. Die Amplitude des Pulsanteils 182 ist geringer als die Amplitude 184 des Pulsanteils 181. Dafür ist die Dauer des Pulsanteils 182 länger als die des Pulsanteils 181. Die Pulsanteile 181 und 182 sind idealerweise so dimensioniert, dass die Ladung, welche durch sie übertragen wird, bei beiden Pulsanteilen 181 und 182 gleich groß ist, d.h. die in Fig. 20 schraffiert eingezeichneten Flächen sind gleich groß. Im Ergebnis wird dadurch durch einen Einzelpuls 180 genauso viel Ladung in das Gewebe eingebracht, wie aus dem Gewebe entnommen wird.

Die in Fig. 20 dargestellte Rechteckform der Einzelpulse 180 stellt eine ideale Form dar. Je nach der Güte der die Einzelpulse 180 erzeugenden Elektronik wird von der idealen Rechteckform abgewichen.

Anstelle von pulsförmigen Reize können auch anders ausgestaltete Reize, z.B. zeitlich kontinuierliche Reizmuster wie etwa ladungsbalanzierte Sinusreize verwendet werden. Die Sinusreize können entweder nur genau eine Sinusperiode oder eine ganzzahlige Anzahl von Sinusperioden dauern, um zu gewährleisten, dass die Reize ladungsbalanziert sind. Die Frequenz der Sinusschwingungen kann im Bereich von 1 bis 150 Hz und insbesondere im Bereich von 60 bis 150 Hz liegen.

Die von den Stimulationseinheiten 112 bis 115 applizierten Reize werden von in oder unter der Haut gelegenen Rezeptoren aufgenommen und an das Nervensystem weitergeleitet. Zu diesen Rezeptoren zählen beispielsweise Merkel-Zellen, Ruffini-Körperchen, Meissner-Körperchen und Haarfollikelrezeptoren, die insbesondere als Rezeptoren für die taktilen Reize wirken. Die vibratorischen Reize zielen vorwiegend auf die Tiefensensibilität ab und werden von in der Haut, den Muskeln, dem Subkutangewebe und/oder den Sehnen des Patienten gelegenen Rezeptoren aufgenommen. Als Rezeptoren für die vibratorischen Reize seien beispielhaft die Vater-Pacini-Körperchen genannt, die Vibrationsempfindungen und Beschleunigungen vermitteln. Die thermischen Reize werden von den Thermorezeptoren der Haut aufgenommen. Dies sind Warmrezeptoren (auch Wärmerezeptoren, Warmsensoren oder Wärmesensoren genannt) und Kaltsensoren (auch Kältesensoren, Kaltrezeptoren oder Kälterezeptoren genannt). In der Haut des Menschen liegen die Kaltsensoren mehr oberflächlich, die Warmrezeptoren etwas tiefer. Die elektrischen transkutanen Reize sind weitgehend unspezifisch und werden von verschiedenen in oder unter der Haut gelegenen Rezeptoren aufgenommen.

Die von den Stimulationselementen 112 bis 115 generierten Reize 120 bis 150 sind derart ausgestaltet, dass sie, wenn sie von den entsprechenden Rezeptoren aufgenommen werden und über die Nervenleitungen zu einer Neuronenpopulation im Gehirn oder Rückenmark mit einer krankhaft synchronen und oszillatorischen Aktivität geleitet werden, in der Neuronenpopulation ein Zurücksetzen der Phase der neuronalen Aktivität der stimulierten Neuronen bewirken. Aufgrund der Mehrzahl von Stimulationselementen 112 bis 115 kann die krankhafte Neuronenpopulation an unterschiedlichen Stellen stimuliert werden. Dies ermöglicht es, die Phase der neuronalen Aktivität der krankhaften Neuronenpopulation an den unterschiedlichen Stimulationsstellen zu unterschiedlichen Zeitpunkten zurückzusetzen. Im Ergebnis wird dadurch die krankhafte Neuronenpopulation, deren Neuronen zuvor synchron und mit gleicher Frequenz und Phase aktiv waren, in mehrere Subpopulationen aufgespalten. Durch eine derartige CR-Neuromodulation kann die Desynchronisation der zuvor krankhaft synchronen Neuronenpopulation bewirkt werden.

Schematisch ist eine mit Hilfe der Stimulationselemente 112 bis 115 durchgeführte CR-Stimulation in Fig. 21 dargestellt. Über die Stimulationselemente 112 bis 115 werden an unterschiedlichen Stellen der Haut des Patienten die jeweiligen Rezeptoren mit taktilen und/oder vibratorischen und/oder thermischen und/oder elektrisch transkutanen Reizen 120 bis 150 stimuliert.

Bei der in Fig. 21 dargestellten Ausgestaltung appliziert jedes der Stimulationselemente 112 bis 115 einen Reiz 120 bis 150 periodisch mit der Frequenz fₛₜᵢₘ = 1 /Tₛₜᵢₘ. Die Frequenz fₛₜᵢₘ kann im Bereich von 1 bis 60 Hz und insbesondere im Bereich von 30 bis 60 Hz oder im Bereich von 1 bis 30 Hz oder im Bereich 1 bis 20 Hz oder im Bereich von 5 bis 20 Hz liegen, kann aber auch kleinere oder größere Werte annehmen. Insbesondere kann die Frequenz fₛₜᵢₘ nahe der mittleren Frequenz der krankhaft rhythmischen Aktivität des Ziel-Netzwerks liegen.

Die Verabreichung der Reize 120 bis 150 über unterschiedliche Stimulationselemente 112 bis 115 erfolgt mit einer zeitlichen Verzögerung τ zwischen den einzelnen Stimulationselementen 112 bis 115 um Tₛₜᵢₘ/4.

Im Fall von N Stimulationselementen kann die zeitliche Verzögerung τ zwischen jeweils zwei aufeinander folgenden Reizen 120 bis 150 beispielsweise im Bereich eines N-tels der Periode 1/fₛₜᵢₘ liegen, d.h. 1/(N x fₛₜᵢₘ) = Tₛₜᵢₘ/N, d.h., insbesondere vergeht zwischen den Startzeitpunkten von zwei aufeinander folgenden Reizen 120 bis 150 die Zeit Tₛₜᵢₘ/N. Von der Vorgabe, dass die zeitliche Verzögerung τ zwischen jeweils zwei aufeinander folgenden Reizen Tₛₜᵢₘ/N beträgt, kann bis zu einem gewissen Grad abgewichen werden. Beispielsweise kann von dem Wert Tₛₜᵢₘ/N für die zeitliche Verzögerung τ um bis zu ± 5%, ± 10% oder ± 20% abgewichen werden. Bei einer derartigen Abweichung wurden noch Stimulationserfolge erzielt, d.h., es konnte noch ein desynchronisierender Effekt beobachtet werden.

Die von den Stimulationselementen 112 bis 115 applizierten Reize 120 bis 150 werden an unterschiedliche Subpopulationen der Neuronenpopulation weitergeleitet (vgl. z.B. Neuronenpopulation 27 mit Subpopulationen 28 bis 31 in Fig. 1) und resetten die Phasen dieser Subpopulationen zu jeweils unterschiedlichen Zeitpunkten, wodurch eine Desynchronisation der gesamten Neuronenpopulation erzielt wird.

Die gezielte Stimulation bestimmter Bereiche des Gehirns oder Rückenmarks wird durch die somatotope Zuordnung von Körperregionen zu diesen Bereichen ermöglicht. Beispielsweise können die Stimulationselemente 112 bis 115 am Fuß, Unterschenkel und Oberschenkel oder aber an der Hand, dem Unterarm und Oberarm des Patienten angebracht werden. Aufgrund der somatotopischen Gliederung der Nervenleitungsbahnen werden durch die an den jeweiligen Stellen applizierten Reize unterschiedliche Neuronen stimuliert. Die somatotope Zuordnung von Hautstellen zu Bereichen des Gehirns ist beispielsweise in A. Benninghoff et al.: "Lehrbuch der Anatomie des Menschen. Dargestellt unter Bevorzugung funktioneller Zusammenhänge. 3. Bd. Nervensystem, Haut und Sinnesorgane", Urban und Schwarzenberg, München 1964, beschrieben.

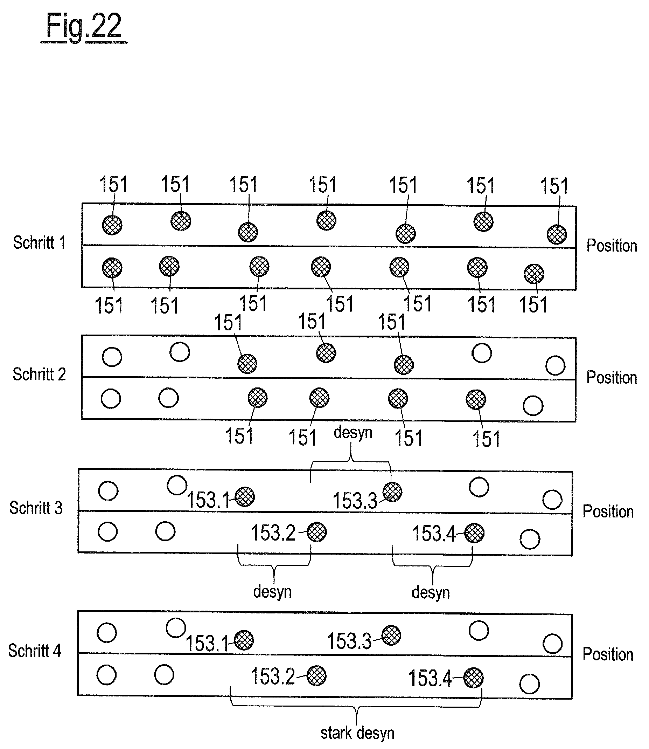

Zur Bestimmung der optimalen Reizparameter für die taktile, vibratorische, thermische und/oder elektrische transkutane CR-Neuromodulation kann die bereits oben erläuterte Eichprozedur durchgeführt werden. Ein Flussdiagramm zur Veranschaulichung des Verfahrensablaufs der Eichung ist in Fig. 22 dargestellt. Für jeden der vier Eichschritte ist dort schematisch die Vorderseite (oben) und Unterseite (unten) des Arms eines Patienten dargestellt. Rechts befindet sich die Hand (nicht dargestellt), links die Schulter (nicht dargestellt). Jeweils für Vorder- und Rückseite des Arms sind in Fig. 22 die Positionen der Stimulationselemente dargestellt. Die Stimulationselemente können beispielsweise mit Klettverschlüssen am Arm des Patienten befestigt werden, wodurch sich die Position der Stimulationselemente leicht verändern lässt.

Im ersten Schritt wird ein erstes Hautareal ausgewählt, welches das erkrankte Körperteil einschließt (also etwas größer ist, damit die tatsächlich benötigte Ausdehnung durch die Selektion der optimalen Einzelreize ermittelt werden kann) bzw. Repräsentationen (z.B. Headsche Zonen) des erkrankten Körperteils bzw. Organs einschließt. Innerhalb des ausgewählten ersten Hautareals werden taktile, vibratorische, thermische und/oder elektrische transkutane erste Reize 151 an den in Fig. 22 gezeigten Positionen appliziert. Dabei wird das zweidimensionale Startareal der Haut gemäß der dem Fachmann bekannten physiologischen somatotopen Abbildungscharakteristika so mit den ersten Reizen 151 abgedeckt, dass die zugehörigen Zielorte im Gehirn, die durch die ersten Reize 151 stimuliert werden, in erster Näherung gleiche räumliche Entfernungen voneinander haben, also äquidistant sind.

Die ersten Reize 151 werden nun von der Steuer- und Analyseeinheit 110 darauf getestet, ob sie die Phase der pathologischen, synchronisierten oszillatorischen Hirnaktivität zurücksetzen können. Dabei werden aus der Vorauswahl an ersten Reizen 151 diejenigen ersten Reize 151 selektiert, welche die Phase der pathologischen, synchronisierten und oszillatorischen Hirnaktivität zurückzusetzen vermögen. Fig. 22 zeigt beispielhaft die dabei ausgewählten ersten Reize 151 im Schritt 2. Die nicht wirksamen, d.h. nicht phasenrücksetzenden und dementsprechend verworfenen ersten Reize 151 sind im Schritt 2 als nicht ausgefüllte Kreise dargestellt.

Sofern alle ersten Reize 151 aus der Startauswahl, d.h. alle im Schritt 1 der Fig. 22 gezeigten ersten Reize 151, die Phase der pathologischen, synchronisierten und oszillatorischen Hirnaktivität zurückzusetzen vermögen, werden diesen ersten Reizen 151 noch zweite Reize ("Randreize") hinzugefügt (in Fig. 22 nicht dargestellt). Die zweiten Reize liegen außerhalb des ersten Hautareals, aber innerhalb eines das erste Hautareal umfassenden zweiten Hautareals. Auch unter den zweiten Reizen werden diejenigen Reize ausgewählt, die die Phase der pathologischen, synchronisierten und oszillatorischen Hirnaktivität zurücksetzen können.

Nachdem im zweiten Schritt die wirksamen, d.h. phasenrücksetzenden Reize selektiert worden sind, ermittelt die Steuer- und Analyseeinheit 110 im dritten Schritt diejenigen dritten Reize 153, die das zugehörige Hirnareal möglichst äquidistant reizen. Dazu wird folgendermaßen vorgegangen. Für den Fall, dass im zweiten Schritt keine zweiten Reize ausgewählt wurden, werden verteilt auf das gesamte wirksame Hautareal neben den außen gelegenen ersten Reizen 151 gemäß dem Fachmann bekannter somatotoper Zuordnung noch wenige weitere Reize ausgewählt, die zwischen den beiden im wirksamen Hautareal außen gelegenen ersten Reizen 151 liegen. Diese Reize bilden die dritten Reize 153. In Fig. 22 sind beispielhaft vier dritte Reize 153.1, 153.2, 153.3 und 153.4 dargestellt. Vorzugsweise sind die Zielorte der dritten Reize 153.1 bis 153.4 im Gehirn oder Rückenmark des Patienten näherungsweise äquidistant.

Sofern im zweiten Schritt zweite Reize ausgewählt wurden, werden die im wirksamen (zweiten) Hautareal außen gelegenen zweiten Reize als "Randreize" der dritten Reize 153 gewählt (entsprechend den Reizen 153.1 und 153.4 aus Fig. 22). Zwischen diesen äußeren zweiten Reizen werden noch weitere dritte Reize verteilt (entsprechend den Reizen 153.2 und 153.3 aus Fig. 22).

Mit den dritten Reize 153.1 bis 153.4 wird jeweils paarweise eine CR-Stimulation durchgeführt, wie sie in Fig. 21 gezeigt ist (in diesem Fall mit nur zwei Kanälen). Zunächst wird das Reizpaar 153.1/ 153.2 getestet. Sofern dieser Test einen Amplitudenanstieg der pathologischen Oszillation (also eine Verstärkung der Synchronisation der das Signal generierenden pathologisch synchronisierten Neuronenpopulation) ergeben sollte, wird das den Reiz 153.2 erzeugende Stimulationselement solange weiter von dem den Reiz 153.1 erzeugenden Stimulationselement weggeschoben, bis die CR-Stimulation mit dem Reizpaar 153.1/153.2 keinen Amplitudenanstieg der pathologischen Oszillation mehr zeigt. Anschließend wird das Reizpaar 153.2/153.3 auf die gleiche Art getestet. Falls erforderlich wird hier das den Reiz 153.3 erzeugende Stimulationselement verschoben, bis bei der CR-Stimulation die Amplitude der pathologischen Oszillation nicht mehr wächst. Danach wird das Reizpaar 153.3/ 153.4 ausgetestet. Dabei kann es vorkommen, dass der äußerste Reiz 153.4 aus dem zweiten Hautareal hinauswandert. In diesem Fall kann gegebenenfalls einer der dritten Reize, z.B. der Reiz 153.3, verworfen werden. Der Test würde dann mit dem Reizpaar 153.2/153.4 durchgeführt.

Im vierten Schritt prüft die Steuer- und Analyseeinheit 110, ob bei Verwendung aller im dritten Schritt bestimmten dritten Reize 153.1 bis 153.4 die zugehörige CR-Stimulation die krankhaft synchrone und oszillatorische neuronale Aktivität der stimulierten Neuronen unterdrückt und insbesondere desynchronisierend wirkt. Dazu wird eine CR-Stimulation durchgeführt, wie sie in Fig. 21 gezeigt ist. Eine solche CR-Stimulation mit allen selektierten dritten Reizen 153.1 bis 153.4 sollte zu einer Abnahme der Amplitude des pathologischen Signals führen, was einer CRinduzierten Desynchronisation der zugrundeliegenden, krankhaft synchronisierten Neuronenpopulation entspricht.

## Patentansprüche

1. Vorrichtung (1) zur Stimulation von Neuronen mit einer krankhaft synchronen und oszillatorischen neuronalen Aktivität, umfassend
- eine nicht-invasive Stimulationseinheit (11) zur Applikation von Reizen (22) an einen Patienten, wobei die Reize (22) Neuronen im Gehirn und/oder Rückenmark des Patienten stimulieren,
- eine Messeinheit (12) zum Aufnehmen von Messsignalen (23), die eine neuronale Aktivität der stimulierten Neuronen wiedergeben, und
- eine Steuer- und Analyseeinheit (10) zur Steuerung der Stimulationseinheit (11) und zur Analyse der Messsignale (23), wobei die Steuer- und Analyseeinheit (10) derart ausgestaltet ist, dass sie
- die Stimulationseinheit (11) derart ansteuert, dass diese erste Reize (34) appliziert, und
- anhand der in Reaktion auf die Applikation der ersten Reize (34) aufgenommenen Messsignale (23) die ersten Reize (34) auswählt, die eine Phasenrücksetzung der krankhaft synchronen und oszillatorischen neuronalen Aktivität der stimulierten Neuronen bewirken,
**dadurch gekennzeichnet, dass** die Steuer- und Analyseeinheit (10) ferner derart ausgestaltet ist, dass sie
- die Stimulationseinheit (11) derart ansteuert, dass diese die ausgewählten ersten Reize (34) oder aus den ausgewählten ersten Reizen (34) generierte Reize (38) zeitversetzt appliziert, indem die Stimulationseinheit (11) zwei von den ausgewählten ersten Reizen (34) und den aus den ausgewählten ersten Reizen (34) generierten Reize (38) jeweils in einer periodischen Sequenz appliziert, wobei die zwei Sequenzen zeitversetzt sind, jedoch einander zeitlich überlappen, und
- anhand der in Reaktion auf die zeitversetzt applizierten Reize (34, 38) aufgenommenen Messsignale (23) prüft, ob die zeitversetzt applizierten Reize (34, 38) die krankhaft synchrone und oszillatorische Aktivität der stimulierten Neuronen unterdrücken.

2. Vorrichtung (1) nach Anspruch 1, wobei die ersten Reize (34) jeweils einen Reizparameter aufweisen, der für alle ersten Reize (34) innerhalb eines ersten Reizparameterbereichs liegt.

3. Vorrichtung (1) nach Anspruch 2, wobei die Steuer- und Analyseeinheit (10) derart ausgestaltet ist, dass sie,
- falls alle applizierten ersten Reize (34) eine Phasenrücksetzung der krankhaft synchronen und oszillatorischen neuronalen Aktivität der stimulierten Neuronen bewirken, die Stimulationseinheit (11) derart ansteuert, dass diese zweite Reize appliziert, wobei die zweiten Reize jeweils einen Reizparameter aufweisen, der für alle zweiten Reize außerhalb des ersten Reizparameterbereichs und innerhalb eines zweiten Reizparameterbereichs liegt, und
- anhand der in Reaktion auf die Applikation der zweiten Reize aufgenommenen Messsignale (23) die zweiten Reize auswählt, die eine Phasenrücksetzung der krankhaft synchronen und oszillatorischen neuronalen Aktivität der stimulierten Neuronen bewirken.

4. Vorrichtung (1) nach Anspruch 3, wobei die Steuer- und Analyseeinheit (10) derart ausgestaltet ist, dass sie
- eine Auswahl dritter Reize (38) bildet, wobei zu den dritten Reizen (38) die im zweiten Reizparameterbereich außen gelegenen ausgewählten zweiten Reize sowie weitere zwischen diesen zweiten Reizen liegende Reize gehören,
- die Stimulationseinheit (11) derart ansteuert, dass diese nur zwei der dritten Reize (38) zeitversetzt appliziert, und
- anhand der in Reaktion auf die zeitversetzt applizierten dritten Reize (38) aufgenommenen Messsignale (23) prüft, ob die zeitversetzt applizierten dritten Reize (38) einen Anstieg der krankhaft synchronen und oszillatorischen Aktivität der Neuronen bewirken.

5. Vorrichtung (1) nach Anspruch 2, wobei die Steuer- und Analyseeinheit (10) derart ausgestaltet ist, dass sie
- eine Auswahl dritter Reize (38) bildet, wobei zu den dritten Reizen (38) die im ersten Reizparameterbereich außen gelegenen ausgewählten ersten Reize (34) sowie weitere zwischen diesen ersten Reizen (34) liegende Reize gehören,
- die Stimulationseinheit (11) derart ansteuert, dass diese nur zwei der dritten Reize (38) zeitversetzt appliziert, und
- anhand der in Reaktion auf die zeitversetzt applizierten dritten Reize (38) aufgenommenen Messsignale (23) prüft, ob die zeitversetzt applizierten dritten Reize (38) einen Anstieg der krankhaft synchronen und oszillatorischen Aktivität der Neuronen bewirken.

6. Vorrichtung (1) nach Anspruch 4 oder 5, wobei die Steuer- und Analyseeinheit (10) derart ausgestaltet ist, dass sie, falls die zwei zeitversetzt applizierten dritten Reize (38) einen Anstieg der krankhaft synchronen und oszillatorischen Aktivität der Neuronen bewirken, den Reizparameter eines der zwei dritten Reiz (38) variiert.

7. Vorrichtung (1) nach Anspruch 6, wobei durch die Variation des Reizparameters des einen der zwei dritten Reize (38) die tonotope oder somatotope Distanz zwischen den zwei dritten Reizen (38) vergrößert wird.

8. Vorrichtung (1) nach einem der vorhergehenden Ansprüche, wobei die ersten Reize (34) derart gewählt werden, dass ihre zugehörigen Zielorte im Gehirn oder Rückenmark des Patienten näherungsweise äquidistant sind.

9. Vorrichtung (1) nach einem der vorhergehenden Ansprüche, wobei die Stimulationseinheit (11) derart ausgestaltet ist, dass sie Reize (22) aus der Gruppe der akustischen, optischen, taktilen, vibratorischen, thermischen und elektrischen transkutanen Reize generiert.

10. Vorrichtung (1) nach Anspruch 9, wobei
- der Reizparameter eine Frequenz im Fall von akustischen Reizen (22) ist und der erste und zweite Reizparameterbereich jeweils ein Frequenzintervall sind,
- der Reizparameter eine Position im Gesichtsfeld des Patienten im Fall von optischen Reizen (22) ist und der erste und zweite Reizparameterbereich jeweils ein Gesichtsfeldbereich sind, und
- der Reizparameter eine Position auf der Haut des Patienten im Fall von taktilen, vibratorischen, thermischen und elektrischen transkutanen Reizen (22) ist und der erste und zweite Reizparameterbereich jeweils ein Hautareal sind.

## Claims

1. An apparatus (1) for stimulating neurons with a pathologically synchronous and oscillatory neural activity, comprising
- a non-invasive stimulation unit (11) for applying stimuli (22) to a patient, wherein the stimuli (22) stimulate neurons in the brain and/or spinal cord of the patient;
- a measuring unit (12) for recording measured signals (23) which reproduce a neural activity of the stimulated neurons; and
- a control and analysis unit (10) for controlling the stimulation unit (11) and for analyzing the measured signals (23), wherein the control and analysis unit (10) is configured such that it
- controls the stimulation unit (11) such that it applies first stimuli (34); and
- selects the first stimuli (34) which effect a phase reset of the pathologically synchronous and oscillatory neural activity of the stimulated neurons with reference to the measured signals (23) recorded in response to the application of the first stimuli (34), **characterized in that** the control and analysis unit (10) is furthermore configured such that it
- controls the stimulation unit (11) such that it applies the selected first stimuli (34) or stimuli (38) generated from the selected first stimuli (34) with a time offset **in that** the stimulation unit (11) applies two of the selected first stimuli (34) and of the stimuli (38) generated from the selected first stimuli (34) in a respective periodic sequence, wherein the two sequences are offset in time, but overlap one another in time; and
- checks with reference to the measured signals (23) recorded in response to the stimuli (34, 38) applied with a time offset whether the stimuli (34, 38) applied with a time offset suppress the pathologically synchronous and oscillatory activity of the stimulated neurons.

2. An apparatus (1) in accordance with claim 1, wherein the first stimuli (34) each have a stimulus parameter which lies within a first stimulus parameter range for all first stimuli (34).

3. An apparatus (1) in accordance with claim 2, wherein the control and analysis unit (10) is configured such that,
- if all the applied first stimuli (34) effect a phase reset of the pathologically synchronous and oscillatory neural activity of the stimulated neurons, it controls the stimulation unit (11) such that it applies these second stimuli, wherein the second stimuli each have a stimulus parameter which lies outside the first stimulus parameter range and within a second stimulus parameter range for all second stimuli; and
- it selects the second stimuli which effect a phase reset of the pathologically synchronous and oscillatory neural activity of the stimulated neurons with reference to the measured signals (23) recorded in response to the application of the second stimuli.

4. An apparatus (1) in accordance with claim 3, wherein the control and analysis unit (10) is configured such that it
- forms a selection of third stimuli (38), wherein the selected second stimuli disposed outwardly in the second stimulus parameter range as well as further stimuli disposed between these second stimuli belong to the third stimuli (38);
- controls the stimulation unit (11) such that it only applies two of the third stimuli (38) with a time offset; and
- checks with reference to the measured signals (23) recorded in response to the third stimuli (38) applied with a time offset whether the third stimuli (38) applied with a time offset effect an increase in the pathologically synchronous and oscillatory activity of the neurons.

5. An apparatus (1) in accordance with claim 2, wherein the control and analysis unit (10) is configured such that it
- forms a selection of third stimuli (38), wherein the selected first stimuli (34) disposed outwardly in the first stimulus parameter range as well as further stimuli disposed between these first stimuli (34) belong to the third stimuli (38);
- controls the stimulation unit (11) such that it only applies two of the third stimuli (38) with a time offset; and
- checks with reference to the measured signals (23) recorded in response to the third stimuli (38) applied with a time offset whether the third stimuli (38) applied with a time offset effect an increase in the pathologically synchronous and oscillatory activity of the neurons.

6. An apparatus (1) in accordance with claim 4 or claim 5, wherein the control and analysis unit (10) is configured such that it varies the stimulus parameter of one of the two third stimuli (38) if the two third stimuli (38) applied with a time offset effect an increase in the pathologically synchronous and oscillatory activity of the neurons.

7. An apparatus (1) in accordance with claim 6, wherein the tonotopic or somatotopic distance between the two third stimuli (38) is enlarged by the variation of the stimulus parameter of the one of the two third stimuli (38).

8. An apparatus (1) in accordance with any one of the preceding claims, wherein the first stimuli (34) are selected such that their associated target sites in the brain or spinal cord of the patient are approximately equidistant.

9. An apparatus (1) in accordance with any one of the preceding claims, wherein the stimulation unit (11) is configured such that it generates stimuli (22) from the group of acoustic, optical, tactile, vibratory, thermal and electrical transcutaneous stimuli.

10. An apparatus (1) in accordance with claim 9, wherein
- the stimulus parameter is a frequency in the event of acoustic stimuli (22) and the first and second stimulus parameter ranges are each a frequency interval;
- the stimulus parameter is a position in the visual field of the patient in the case of optical stimuli (22) and the first and second stimulus parameter ranges are each a visual field region; and
- the stimulus parameter is a position on the skin of the patient in the case of tactile, vibratory, thermal and electrical transcutaneous stimuli (22) and the first and second stimulus parameter ranges are each a skin area.

## Revendications

1. Dispositif (1) pour la stimulation de neurones avec une activité neuronale pathologique synchrone et oscillatoire, incluant
- une unité de stimulation non-invasive (11) pour l'application d'excitations (22) à un patient, les excitations (22) stimulant des neurones dans le cerveau et/ou la moelle épinière du patient,
- une unité de mesure (12) pour enregistrer des signaux de mesure (23) qui reproduisent une activité neuronale des neurones stimulés, et
- une unité de commande et d'analyse (10) pour la commande de l'unité de stimulation (11) et pour l'analyse des signaux de mesure (23), dans lequel l'unité de commande et d'analyse (10) est conçue de telle façon
- qu'elle pilote l'unité de stimulation (11) de telle manière que celle-ci applique des premières excitations (34), et
- au moyen des signaux de mesure (23) enregistrés en réaction à l'application des premières excitations (34), qu'elle sélectionne les premières excitations (34) qui provoquent une remise en phase de l'activité neuronale pathologique synchrone et oscillatoire des neurones stimulés,
**caractérisé en ce que** l'unité de commande et d'analyse (10) est en outre conçue de telle façon
- qu'elle pilote l'unité de stimulation (11) de telle manière que celle-ci applique les premières excitations sélectionnées (34) ou bien des excitations (38) générées à partir des premières excitations sélectionnées, de manière décalée dans le temps, du fait que l'unité de stimulation (11) applique respectivement dans une séquence périodique deux excitations (38) parmi les premières excitations sélectionnées (34) et les excitations (38) générées à partir des premières excitations sélectionnées (34), dans lequel les deux séquences sont décalées dans le temps, mais se chevauchent temporellement mutuellement, et
- au moyen des signaux de mesure enregistrés en réaction aux excitations appliquées de manière décalée dans le temps (34, 38), qu'elle vérifie si les excitations appliquées de manière décalée dans le temps (34, 38) suppriment l'activité pathologique synchrone et oscillatoire des neurones stimulés.

2. Dispositif (1) selon la revendication 1, dans lequel les premières excitations (34) présentent chacune un paramètre d'excitation qui tombe à l'intérieur d'une première plage de paramètre d'excitation pour toutes les premières excitations (34).

3. Dispositif (1) selon la revendication 2, dans lequel l'unité de commande et d'analyse (10) est conçue de telle façon
- dans le cas où toutes les premières excitations appliquées (34) provoquent une remise en phase de l'activité neuronale pathologique synchrone et oscillatoire des neurones stimulés, qu'elle pilote l'unité de stimulation (11) de telle façon que celle-ci applique des secondes excitations, lesdites secondes excitations présentant chacune un paramètre d'excitation qui, pour toutes les secondes excitations, tombe à l'extérieur de la première plage de paramètre d'excitation et à l'intérieur d'une seconde plage de paramètre d'excitation, et
- au moyen des signaux de mesure (23) enregistrés en réaction à l'application des secondes excitations, qu'elle sélectionne les secondes excitations qui provoquent une remise en phase de l'activité neuronale pathologique synchrone et oscillatoire des neurones stimulés.

4. Dispositif (1) selon la revendication 3, dans lequel l'unité de commande et d'analyse (10) est conçue de telle façon
- qu'elle forme une sélection de troisièmes excitations (38), troisièmes excitations (38) auxquelles appartiennent les secondes excitations sélectionnées qui se trouvent à l'extérieur dans la seconde plage de paramètre d'excitation ainsi que d'autres secondes excitations entre celles-ci,
- qu'elle pilote l'unité de stimulation (11) de telle façon que celle-ci applique uniquement deux des troisièmes excitations (38) de manière décalée dans le temps, et
- au moyen des signaux de mesure (23) enregistrés en réaction aux troisièmes excitations (38) appliquées de manière décalée dans le temps, qu'elle vérifie si les troisièmes excitations (38) appliquées de manière décalée dans le temps provoquent une augmentation de l'activité pathologique synchrone et oscillatoire des neurones.

5. Dispositif (1) selon la revendication 2, dans lequel l'unité de commande et d'analyse (10) est conçue de telle façon
- qu'elle forme une sélection de troisièmes excitations (38), troisièmes excitations (38) auxquelles appartiennent les premières excitations (34) sélectionnées disposées à l'extérieur dans la première plage de paramètre d'excitation, ainsi que d'autres excitations situées entre ces premières excitations (34),
- qu'elle pilote l'unité de stimulation (11) de telle façon que celle-ci applique seulement deux des troisièmes excitations (38) de manière décalée dans le temps, et
- au moyen des signaux de mesure (23) enregistrés en réaction aux troisièmes excitations (38) appliquées de manière décalée dans le temps, qu'elle vérifie si les troisièmes excitations (38) appliquées de manière décalée dans le temps provoquent une augmentation de l'activité pathologique synchrone et oscillatoire des neurones.

6. Dispositif (1) selon la revendication 4 ou 5, dans lequel l'unité de commande et d'analyse (10) est conçue de telle façon que, dans le cas où les deux troisièmes excitations (38) appliquées de manière décalée dans le temps provoquent une augmentation de l'activité pathologique synchrone et oscillatoire des neurones, elle fait varier le paramètre d'excitation de l'une des deux troisièmes excitations (38).

7. Dispositif (1) selon la revendication 6, dans lequel la distance tonotope ou somatotope entre les deux troisièmes excitations (38) est agrandie par la variation du paramètre d'excitation de l'une des deux troisièmes excitations (38).

8. Dispositif (1) selon l'une des revendications précédentes, dans lequel les premières excitations (34) sont sélectionnées de telle façon que leurs cibles locales associées dans le cerveau ou dans la moelle épinière du patient sont approximativement équidistantes.

9. Dispositif (1) selon l'une des revendications précédentes, dans lequel l'unité de stimulation (11) est conçue de telle façon qu'elle génère des excitations (22) parmi le groupe des excitations acoustiques, optiques, tactiles, vibratoires, thermiques et électriques transcutanées.

10. Dispositif (1) selon la revendication 9, dans lequel
- le paramètre d'excitation est une fréquence dans le cas d'excitations acoustiques (22) et la première et la seconde plage de paramètre d'excitation sont respectivement un intervalle de fréquence,
- le paramètre d'excitation est une position dans la zone du visage du patient dans le cas d'excitations optiques (22), et la première et la seconde plage de paramètre d'excitation sont respectivement une plage dans la zone du visage, et
- le paramètre d'excitation est une position sur la peau du patient dans le cas d'excitations tactiles, vibratoires, thermiques et électriques transcutanées (22), et la première et la seconde plage de paramètre d'excitation sont respectivement une zone de la peau.
